# EUROPEAN PATENT APPLICATION

(11) **EP 1 719 554 A2**
(43) Date of publication of application: **08.11.2006**
(21) Application number: 06252409.5
(22) Date of filing: 05.05.2006
(51) Int. Cl.: B01J 13/18, B01J 13/02, B01J 13/10, B01J 13/16

(54) **Encapsulated fragrance materials and methods for making same**

(30) Priority: 06.05.2005 US 123898; 03.04.2006 US 278440
(71) Applicant: INTERNATIONAL FLAVORS & FRAGRANCES, INC., New York New York 10019 (US)
(72) Inventor: Pluyter, Johan Gerwin Ledewijk, Middletown New Jersey, 07748-2855 (US); Anastasiou, Theodore James, Red Bank New Jersey, 07701 (US)
(74) Representative: Lawrence, John

(57) **Abstract**

The present invention is directed to novel capsules containing active materials and methods for making capsules with enhanced performance and stability. The capsules are well suited for use in personal care applications, laundry products and perfume and fragrance products.

## Description

### Related Application

This application is a continuation-in-part of the U.S. application serial number 11/123,898, filed May 6, 2005, now pending.

### Field of the Invention

The present invention is directed to novel capsules containing active materials and to methods for making capsules with enhanced performance and stability. The capsules are well suited for use in personal care applications, laundry products and perfume and fragrance products.

### Background of the Invention

Encapsulation of active materials, such as fragrances, is well known in the art. Encapsulation provides advantages to the fragrance product including the protection of the fragrance in the capsule core by a shell until the fragrance is intended to be delivered. In particular, capsules are often designed to deliver their contents at a desired time by the capsule shell being compromised at the desired time.

The capsule shell can be compromised by various factors such as temperature so that the contents are delivered when the capsule begins to melt. Alternatively the capsules can be compromised by physical forces, such as crushing, or other methods that compromise the integrity of the capsule. Additionally, the capsule contents may be delivered via diffusion through the capsule wall during a desired time interval.

It is obviously not desired that the core be released from the shell prematurely. Often, the capsule shell is somewhat permeable to the core contents when stored under certain conditions. This is particularly the case when many capsule types, such as those having aminoplast or cross-linked gelatin walls, are stored in aqueous bases, particularly those containing surfactants. In these cases, although the capsule shell is intact, the fragrance is removed from the core over time in a leaching process. The overall leaching mechanism may be viewed as a diffusion process, with transfer occurring from the capsule core to the aqueous media, followed by transfer to or solubilization into the surfactant micelles or vesicles. With normal surfactant concentrations of between 4 and 30% in consumer products, as compared to fragrance levels of 0.3 to 1%, it is clear that the partitioning favors absorption by the surfactant over time.

In order to enhance the effectiveness of the fragrance materials for the user, various technologies have been employed to enhance the delivery of the fragrance materials at the desired time. One widely used technology is encapsulation of the fragrance material in a protective coating. Frequently the protective coating is a polymeric material. The polymeric material is used to protect the fragrance material from evaporation, reaction, oxidation or otherwise dissipating prior to use. A brief overview of polymeric encapsulated fragrance materials is disclosed in the following U.S. Patents: U.S. Patent No. 4,081,384 discloses a softener or anti-stat core coated by a polycondensate suitable for use in a fabric conditioner; U.S. Patent No. 5,112,688 discloses selected fragrance materials having the proper volatility to be coated by coacervation with micro particles in a wall that can be activated for use in fabric conditioning; U.S. Patent No. 5,145,842 discloses a solid core of a fatty alcohol, ester, or other solid plus a fragrance coated by an aminoplast shell; and U.S. Patent No. 6,248,703 discloses various agents including fragrance in an aminoplast shell that is included in an extruded bar soap.

While encapsulation of fragrance in a polymeric shell can help prevent fragrance degradation and loss, it is often not sufficient to significantly improve fragrance performance in consumer products. Therefore, methods of aiding the deposition of encapsulated fragrances have been disclosed. U.S. Patent No. 4,234,627 discloses a liquid fragrance coated with an aminoplast shell further coated by a water insoluble meltable cationic coating in order to improve the deposition of capsules from fabric conditioners. U.S. Patent No. 6,194,375 discloses the use of hydrolyzed polyvinyl alcohol to aid deposition of fragrance-polymer particles from wash products. U.S. Patent No. 6,329,057 discloses use of materials having free hydroxy groups or pendant cationic groups to aid in the deposition of fragranced solid particles from consumer products.

Despite the above teaching and previous encapsulation technologies, there is an ongoing need to develop fragrance systems which are designed to retain the fragrance with minimal losses until it is needed and then be able to deliver the fragrance at the appropriate time.

### Sumnary of the Invention

One embodiment of the invention is directed to a polymer encapsulated active material wherein said polymeric material comprises an amine-containing and/or an amine-generating polymer or mixtures thereof and a crosslinker to provide enhanced deposition.

Another embodiment of the invention is directed to a method for preparing a polymeric encapsulated active material wherein the polymeric material comprises amine-containing polymers, amine-generating polymers and mixtures of both polymers and a crosslinker to provide enhanced deposition.

In a further embodiment of the invention a process is disclosed for improving the performance and stability of encapsulated active materials by catalyzing the curing crosslinking reaction with acids, metal salts and mixtures thereof during capsule formation.

In yet a further embodiment of the invention a secondary crosslinker is added to the encapsulated active material thereby modifying the capsule surface to provide enhanced leaching and deposition properties.

In yet another embodiment the amine containing and/or generating polymers can be applied in a multi-shell morphology around any existing capsules of any wall chemistry, so that each of the shells may be comprised of different wall chemistries.

These and other embodiments of the present invention will be apparent by reading the following specification.

According to another embodiment of the invention there is provided a capsule particle comprising an active material; said active material encapsulated by a polymeric material to provide a polymer encapsulated material wherein said polymeric material comprises an amine-containing and/or amine-generating polymer with primary and/or secondary amine groups or mixtures thereof and a cross-linker.

The active material may be selected from the group consisting of fragrances, flavoring agents, fungicide, brighteners, antistatic agents, wrinkle control agents, fabric softener actives, hard surface cleaning actives, skin and/or hair conditioning agents, antimicrobial actives, UV protection agents, insect repellants, animal/vermin repellants, flame retardants, and mixtures thereof.

The said active material may be a fragrance.

The composition, capsule, or active material, may further comprise a malodour counteractant composition. The malodour counteractant composition may be selected from the group consisting of uncomplexed cyclodextrin; odor blockers; reactive aldehydes; flavanoids; zeolites; activated carbon; and mixtures thereof.

The amine-containing polymer may be selected from the group consisting of polyvinyl amines, polyvinyl formamides, polyallyl amines, proteins such as gelatin, zein, albumen, polysaccharides and mixtures thereof.

The polymeric material may be of the general formula: 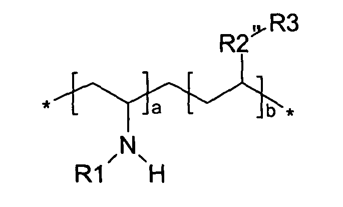 wherein R1 is H, CH₃, (C=O)H, alkylene, alkylene with unsaturated C-C bonds, CH₂-CROH, (C=O) -NH-R, (C=O) - (CH₂) n-OH, (C=O) -R, (CH₂) n-E, - (CH₂-CH(C=O) )n-XR, -(CH₂)n-COOH, -(CH₂)n-NH₂, -CH₂)n-(C=O)NH₂, E is an electrophilic group; wherein a and b are integers or averages (real numbers) from about 100-25,000, R is a saturated or unsaturated alkane, dialkylsiloxy, dialkyloxy, aryl, alkylated aryl, and that may further contain a cyano, OH, COOH, NH₂, NHR, sulfonate, sulphate, -NH₂, quaternized amines, thiols, aldehyde, alkoxy, pyrrolidone, pyridine, imidazol, imidazolinium halide, guanidine, phosphate, monosaccharide, oligo or polysaccharide;
R2 can be absent or the functional group selected from the group consisting of -COO-, -(C=O)-, -O-, -S-, -NH-(C=O)-, -NR1-, dialkylsiloxy, dialkyloxy, phenylene, naphthalene, alkyleneoxy; and
R3 can be equal to or selected from the same group as R1.
The polymeric material may be of the general formula: 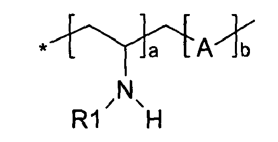 wherein A is an aminal, hydrolyzed or non-hydrolyzed maleic anhydride, vinyl pyrrolidine, vinyl pyridine, vinyl pyridine-N-oxide, methylated vinyl pyridine, vinyl naphthalene, vinyl naphthalene-sulfonate.
The aminal may be of the general formula: 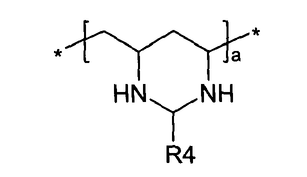 wherein R4 is H, CH₃, (C=O)H, alkylene, alkylene with unsaturated C-C bonds, CH₂-CROH, (C=O)-NH-R, (C=O)-(CH₂)n-OH, (C=O)-R, (CH₂)n-E, -(CH₂-CH (C=O))n-XR, -(CH₂)n-COOH, (CH₂)n-NH₂, -CH₂)n-(C=O)NH₂ and E is an electrophilic group; wherein a and b are integers or averages (real numbers) from about 100-25,000, R is a saturated or unsaturated alkane, dialkylsiloxy, dialkyloxy, aryl, alkylated aryl, and that may further contain a cyano, OH, COOH, NH₂, NHR, sulfonate, sulphate, -NH₂, quaternized amines, thiols, aldehyde, alkoxy, pyrrolidone, pyridine, imidazol, imidazolinium halide, guanidine, phosphate, monosaccharide, oligo or polysaccharide.

The amine-generating polymer may comprise a copolymer or polymer comprising vinyl formamides and/or comprise functional groups selected from the group consisting of imines, amides, enamines, N-nitroso, urea, urethane, ion-paired amine salts, oximes, azo, azoxy, hydrazo and mixtures thereof.

The crosslinker may be selected from the group consisting of aminoplasts, aldehydes such as formaldehyde and acetaldehyde, dialdehydes such as glutaraldehyde, epoxy, active oxygen such as ozone and OH radicals, poly-substituted carboxylic acids and derivatives such as acid chlorides, anyhydrides, isocyanates, diketones, halide-substituted, sulfonyl chloride-based organics, inorganic crosslinkers such as Ca²⁺, organics capable of forming azo, azoxy and hydrazo bonds, lactones and lactams, thionyl chloride, phosgene, tannin/tannic acid, polyphenols, free radical crosslinkers such as benzoyl peroxide, sodium persulfate, azoisobutylnitrile (AIBN) and mixtures thereof.

The polymer may undergo crosslinking by radiation.

The fragrance materials may have greater than about 60 weight percent with ClogP values of equal to or greater than about 3.3.

The capsule particle may additionally comprise a solvent, which may have with logP values of greater than 3.3.

The solvent material may be selected from the group consisting of triglyceride oil, mono and diglycerides, mineral oil, silicone oil, diethyl phtalates, polyalpha olefins, isopropyl myristate and mixtures thereof.

The active material may have from about 10 to about 50 weight percent of the composition.

The capsule particle may be endcapped with mono-functional amino-formaldehyde adducts and/or mono-functional aldehydes such as acetaldehyde and benzaldehyde.

The polymer encapsulated material may be further coated with a cationically charged polymer.

The capsule particle may be incorporated into a product selected from the group consisting of a personal care, fabric care and cleaning products.

The personal care product may be selected from the group consisting of hair shampoos, hair rinses, hair colors and dyes, bar soaps, and body washes.

According to another embodiment of the invention, a method of encapsulating an active material comprises:
providing polymeric material comprising an amine containing polymer and/or an amine-generating polymer or mixtures thereof and a primary crosslinker to completion;
encapsulating the active material with the polymeric material to form a polymer encapsulated material;
an optional step of adding a secondary crosslinker to the reacted polymer encapsulated material in an amount sufficient to modify the capsule surface;
an optional step of adding an amine containing and/or amine generating polymer to the encapsulated material to provide a multi-shell morphology around the encapsulated material; and
providing the polymer encapsulated material to a product selected from the group consisting of a personal care, fabric care and cleaning products.

The active material may be selected from the group consisting of fragrances, flavoring agents, fungicide, brighteners, antistatic agents, wrinkle control agents, fabric softener actives, hard surface cleaning actives, skin and/or hair conditioning agents, antimicrobial actives, UV protection agents, insect repellants, animal/vermin repellants, flame retardants, and mixtures thereof.

The active material may comprise a fragrance having greater than about 60 weight percent with clogP values of greater than about 3.3.

The amine-containing polymer may be selected from the group consisting of polyvinyl amines, polyvinyl formamides, polyallyl amines, proteins such as gelatin, zein, albumen, polysaccharides and mixtures thereof.

The amine-generating polymer may be a copolymer or polymer comprising vinyl formamides and/or comprise functional groups selected from the group consisting of imines, amides, enamines, N-nitroso, urea, urethane, ion-paired amine salts, oximes, azo, azoxy, hydrazo and mixtures thereof.

The primary and secondary crosslinker may be selected from the group consisting of aminoplasts, aldehydes such as formaldehyde and acetaldehyde, dialdehydes such as glutaraldehyde, epoxy, active oxygen such as ozone and OH radicals, poly-substituted carboxylic acids and derivatives such as acid chlorides, anyhydrides, isocyanates, diketones, halide-substituted, sulfonyl chloride-based organics, inorganic crosslinkers such as Ca²⁺, organics capable of forming azo, azoxy and hydrazo bonds, lactones and lactams, thionyl chloride, phosgene, tannin/tannic acid, polyphenols, free radical crosslinkers such as benzoyl peroxide, sodium persulfate, azoisobutylnitrile (AIBN) and mixtures thereof.

The polymer may undergo crosslinking by radiation.

The polymer encapsulated material may be further coated with a cationically charged polymer.

According to another embodiment of the invention a process for preparing an encapsulated active material comprises the steps of:
i. reacting a polymer and a primary crosslinker to completion in the presence of a catalyst in an amount sufficient to adjust the pH to a value from about 3 to about 10.
ii. forming a crosslinked network of the polymer and the primary crosslinker;
iii. admixing an active material to the reactant mixture; and
iv. encapsulating the active material with the polymer to form a polymer encapsulated material; and
v. an optional step of adding a secondary crosslinker to the reacted polymer encapsulated material thereby modifying the capsule surface
vi. an optional step of adding an amine containing and/or amine generating polymer to the encapsulated material to provide a multi-shell morphology around the encapsulated material.

The active material may be selected from the group consisting of fragrances, flavoring agents, fungicide, brighteners, antistatic agents, wrinkle control agents, fabric softener actives, hard surface cleaning actives, skin and/or hair conditioning agents, antimicrobial actives, UV protection agents, insect repellants, animal/vermin repellants, flame retardants, and mixtures thereof.

The active material may be a fragrance.

The catalyst may be selected from the group consisting of hydrochloric (HCl)acid, sulfuric acid, phosphoric acid, para-toluenesulfonic acid (pTSA), acetic acid, glycolic acid, lactic acid, benzoic acid, citric acid, maleic acid, ammonium chloride, aluminum nitrate, aluminum sulfate, magnesium bromide, magnesium chloride, magnesium nitrate, zinc bromide, zinc iodide, zirconyl nitrate and mixtures thereof.

The primary and secondary crosslinker may be selected from the group consisting of aminoplasts, aldehydes such as formaldehyde and acetaldehyde, dialdehydes such as glutaraldehyde, epoxy, active oxygen such as ozone and OH radicals, poly-substituted carboxylic acids and derivatives such as acid chlorides, anyhydrides, isocyanates, diketones, halide-substituted, sulfonyl chloride-based organics, inorganic crosslinkers such as Ca²⁺, organics capable of forming azo, azoxy and hydrazo bonds, lactones and lactams, thionyl chloride, phosgene, tannin/tannic acid, polyphenols, free radical crosslinkers such as benzoyl peroxide, sodium persulfate, azoisobutylnitrile (AIBN) and mixtures thereof.

The polymeric material may undergo crosslinking by radiation.

The polymer may be an acrylamide-acrylic copolymer having a molecular weight in the range of from 500 - 5,000,000; and the weight ratio of acrylic acid monomeric units:acrylamide monomeric units is from 30:1 to 1:30 and wherein the weight ratio of primary crosslinker:acrylamide-acrylic acid copolymer is in the range of from 30:1 to 1:30.

The mole ratio of acrylic acid monomeric units:acrylamide monomeric units may be from 7:3 to 3:7.

The weight ratio of primary crosslinker:acrylamide-acrylic acid copolymer may be in the range of from 14:1 to 1:14.

The weight ratio of primary crosslinker:acrylamide-acrylic acid copolymer may be in the range of from 7:1 to 1:7.

The acrylamide-acrylic acid copolymer may have a molecular weight in the range of from 10,000 to 100,000.

The polymer may be an amine-containing polymer and/or amine-generating polymer having a molecular weight in the range of from 5,000 to 1,000,000 and the weight ratio of primary crosslinker: amine-containing polymer is in the range of from 30:1 to 1:30.

The amine-containing polymer may be selected from the group consisting of polyvinyl amines, polyvinyl formamides, polyallyl amines, proteins such as gelatin, zein, albumen, polysaccharides and mixtures thereof.

The amine-generating polymer may be a copolymer or polymer comprising vinyl formamides and/or comprise functional groups selected from the group consisting of imines, amides, enamines, N-nitroso, urea, urethane, ion-paired amine salts, oximes, azo, azoxy, hydrazo and mixtures thereof.

The primary and secondary crosslinker may be selected from the group consisting of aminoplasts, aldehydes such as formaldehyde and acetaldehyde, dialdehydes such as glutaraldehyde, epoxy, active oxygen such as ozone and OH radicals, poly-substituted carboxylic acids and derivatives such as acid chlorides, anyhydrides, isocyanates, diketones, halide-substituted, sulfonyl chloride-based organics, inorganic crosslinkers such as Ca²⁺, organics capable of forming azo, azoxy and hydrazo bonds, lactones and lactams, thionyl chloride, phosgene, tannin/tannic acid, polyphenols, free radical crosslinkers such as benzoyl peroxide, sodium persulfate, azoisobutylnitrile (AIBN)and mixtures thereof.

The polymeric material may undergo crosslinking by radiation.

The weight ratio of primary crosslinker: amine-containing polymer may be in the range of from 14:1 to 1:14.

The weight ratio of primary crosslinker: amine-containing polymer may be in the range of from 7:1 to 1:7.

The polymeric material may further comprise polymers with reactive groups selected from the group consisting of COOH, OH and SH.

The polymers with reactive groups may be selected from the group consisting of polyacrylates containing acrylic acid copolymers, polyvinyl alcohol, polysaccharides and cysteine.

According to another embodiment of the invention there is provided a capsule particle comprising an active material; said active material encapsulated by a polymeric material wherein said polymeric material is selected from the group consisting of an amine-containing polymer, an amine-generating polymer, mixtures thereof and a cross-linker to provide a polymer encapsulated material.

The amine-containing polymers may comprise primary amine groups.

The amine-containing polymers may comprise secondary amine groups.

The amine-containing polymers may comprise a mixture of primary and secondary amine groups.

The polymeric material may further comprise polymers with reactive groups selected from the group consisting of COOH, OH, SH and mixtures thereof.

The polymers with reactive groups may be selected from the group consisting of polyacrylates containing acrylic acid copolymers, polyvinyl alcohol, polysaccharides, cysteine and mixtures thereof.

The active material may be selected from the group consisting of fragrances, flavoring agents, fungicide, brighteners, antistatic agents, wrinkle control agents, fabric softener actives, hard surface cleaning actives, skin conditioning agents, hair conditioning agents, antimicrobial actives, UV protection agents, insect repellants, animal/vermin repellants, flame retardants and mixtures thereof.

The active material may be a fragrance.

The composition may further comprise a malodour counteractant composition.

The malodour counteractant composition may be selected from the group consisting of uncomplexed cyclodextrin, odor blockers, reactive aldehydes, flavanoids, zeolites, activated carbon and mixtures thereof.

The amine-containing polymer may be selected from the group consisting of polyvinyl amines, polyvinyl formamides, polyallyl amines, gelatin, zein, albumen, polysaccharides and mixtures thereof.

The polymeric material may have the general formula: 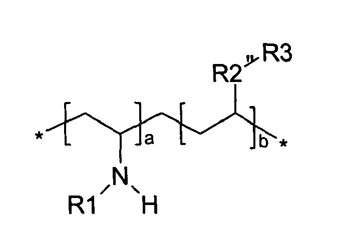 wherein R1 is H, CH₃, (C=O)H, alkylene, alkylene with unsaturated C-C bonds, CH₂-CROH, (C=O) -NH-R, (C=O)-(CH₂)n-OH, (C=O) -R, (CH₂)n-E, -(CH₂-CH(C=O))n-XR, -(CH₂)n-COOH, -(CH₂)n-NH₂, -CH₂)n-(C=O)NH₂, E is an electrophilic group; wherein a and b are integers or averages (real numbers) from about 100-25,000, R is a saturated or unsaturated alkane, dialkylsiloxy, dialkyloxy, aryl, alkylated aryl, and that may further contain a cyano, OH, COOH, NH₂, NHR, sulfonate, sulphate, -NH₂, quaternized amines, thiols, aldehyde, alkoxy, pyrrolidone, pyridine, imidazol, imidazolinium halide, guanidine, phosphate, monosaccharide, oligo or polysaccharide;
R2 can be absent or the functional group selected from the group consisting of -COO-, -(C=O)-, -O-, -S-, -NH-(C=O)-, -NR1-, dialkylsiloxy, dialkyloxy, phenylene, naphthalene, alkyleneoxy; and
R3 can be equal to or selected from the same group as R1.
The polymeric material may be of the general formula: 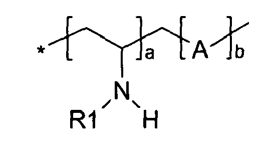 wherein A is an aminal, hydrolyzed or non-hydrolyzed maleic anhydride, vinyl pyrrolidine, vinyl pyridine, vinyl pyridine-N-oxide, methylated vinyl pyridine, vinyl naphthalene, vinyl naphthalene-sulfonate.
The aminal may be of the general formula: 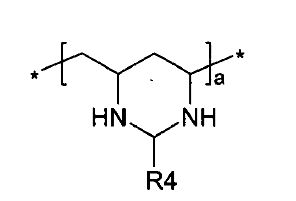 wherein R4 is H, CH₃, (C=O)H, alkylene, alkylene with unsaturated C-C bonds, CH₂-CROH, (C=O)-NH-R, (C=O)-(CH₂)n-OH, (C=O) -R, (CH₂)n-E, -(CH₂-CH(C=O))n-XR, -(CH₂)n-COOH, (CH₂)n-NH₂, -CH₂)n-(C=O)NH₂ and E is an electrophilic group; wherein a is an integer or average (real number) from about 100-25,000, R is a saturated or unsaturated alkane, dialkylsiloxy, dialkyloxy, aryl, alkylated aryl, and that may further contain a cyano, OH, COOH, NH₂, NHR, sulfonate, sulphate, -NH₂, quaternized amines, thiols, aldehyde, alkoxy, pyrrolidone, pyridine, imidazol, imidazolinium halide, guanidine, phosphate, monosaccharide, oligosaccharide and polysaccharide.

The amine-generating polymer may be a polymer comprising vinyl formamides.

The polymer may further comprise functional groups selected from the group consisting of imines, amides, enamines, N-nitroso, urea, urethane, ion-paired amine salts, oximes, azo, azoxy, hydrazo groups and mixtures thereof.

The crosslinker may be selected from the group consisting of aminoplasts, aldehydes, dialdehydes, epoxy, active oxygen, poly-substituted carboxylic acids and derivatives thereof, diketones, halide-substituted, sulfonyl chloride-based organics, inorganic crosslinkers, organic crosslinkers capable of forming azo, azoxy and hydrazo bonds, lactones, lactams, thionyl chloride, phosgene, tannin/tannic acid, polyphenols, free radical crosslinkers and mixtures thereof.

The crosslinker may comprise an aldehyde selected from the group consisting of formaldehyde, acetaldehyde and mixtures thereof.

The crosslinker may be glutaraldehyde.

The crosslinker may comprise an active oxygen selected from the group consisting of ozone, OH radicals and mixtures thereof.

The crosslinker may comprise a poly-substituted carboxylic acids and derivatives thereof selected from the group consisting of acid chlorides, anyhydrides, isocyanates and mixtures thereof.

The crosslinker may comprise a calcium ion (Ca²⁺).

The crosslinker may be a free radical selected from the group consisting of benzoyl peroxide, sodium persulfate, azoisobutylnitrile (AIBN) and mixtures thereof.

The polymer may undergo crosslinking by radiation.

The fragrance materials may have greater than about 60 weight percent with ClogP values of equal to or greater than about 3.3.

The capsule particle may further comprise a solvent which may have logP values of greater than about 3.3.

The solvent material may be selected from the group consisting of triglyceride oil, monoglycerides, diglycerides, mineral oil, silicone oil, diethyl phtalates, polyalpha olefins, isopropyl myristate and mixtures thereof.

The active material may be from about 10 to about 50 weight percent of the composition.

The capsule particle may be endcapped with mono-functional aldehydes selected from the group consisting of acetaldehyde, benzaldehyde and mixtures thereof.

The polymer encapsulated material may be further coated with a cationically charged polymer.

The capsule particle may be incorporated into a product selected from the group consisting of a personal care, fabric care and cleaning products.

The personal care product may be selected from the group consisting of hair shampoos, hair rinses, hair colors and dyes, bar soaps, and body washes.

According to another aspect of the invention a method of encapsulating an active material comprises:
providing polymeric material wherein said polymeric material is selected from the group consisting of amine-containing polymer, amine generating polymers and mixtures thereof and a primary crosslinker to completion;
encapsulating the active material with the polymeric material to form a polymer encapsulated material;
an optional step of adding a secondary crosslinker to the reacted polymer encapsulated material in an amount sufficient to modify the capsule surface;
an optional step of adding a polymeric material selected from the group consisting of an amine-containing polymer, an amine-generating polymer and mixtures thereof to the encapsulated material to provide a multi-shell morphology around the encapsulated material; and
providing the polymer encapsulated material to a product selected from the group consisting of a personal care, fabric care and cleaning products.

The polymeric material may further comprise polymers with reactive groups selected from the group consisting of COOH, OH, SH and mixtures thereof.

The polymers with reactive groups may be selected from the group consisting of polyacrylates containing acrylic acid copolymers, polyvinyl alcohol, polysaccharides, cysteine and mixtures thereof

The active material may be selected from the group consisting of fragrances, flavoring agents, fungicide, brighteners, antistatic agents, wrinkle control agents, fabric softener actives, hard surface cleaning actives, skin and/or hair conditioning agents, antimicrobial actives, UV protection agents, insect repellants, animal/vermin repellants, flame retardants and mixtures thereof.

The active material may be a fragrance having greater than about 60 weight percent with clogP values of greater than about 3.3.

The amine-containing polymer may be selected from the group consisting of polyvinyl amines, polyvinyl formamides, polyallyl amines, gelatin, zein, albumen, polysaccharides and mixtures thereof.

The amine-generating polymer may be polymer comprising vinyl formamides.

The amine-generating polymer may further comprise functional groups selected from the group consisting of imines, amides, enamines, N-nitroso, urea, urethane, ion-paired amine salts, oximes, azo, azoxy, hydrazo and mixtures thereof.

The crosslinker may be selected from the group consisting of aminoplasts, aldehydes such as formaldehyde and acetaldehyde, dialdehydes, epoxy, active oxygen, poly-substituted carboxylic acids and derivatives thereof, diketones, halide-substituted, sulfonyl chloride-based organics, inorganic crosslinkers, organics capable of forming azo, azoxy and hydrazo bonds, lactones, lactams, thionyl chloride, phosgene, tannin/tannic acid, polyphenols, free radical crosslinkers and mixtures thereof.

The crosslinker may be an aldehyde selected from the group consisting of formaldehyde, acetaldehyde and mixtures thereof.

The crosslinker may be glutaraldehyde.

The crosslinker may be active oxygen selected from the group consisting of ozone, OH radicals and mixtures thereof.

The crosslinker may be a poly-substituted carboxylic acids and derivatives thereof selected from the group consisting of acid chlorides, anyhydrides, isocyanates and mixtures thereof.

The crosslinker may be a calcium ion (Ca²⁺).

The crosslinker may be a free radical selected from the group consisting of benzoyl peroxide, sodium persulfate, azoisobutylnitrile (AIBN) and mixtures thereof.

The polymer may undergo crosslinking by radiation.

The polymer encapsulated material may be further coated with a cationically charged polymer.

According to another embodiment of the invention a process for preparing an encapsulated active material comprises the steps of:
vii. reacting a polymer and a primary crosslinker to completion in the presence of a catalyst in an amount sufficient to adjust the pH to a value from about 3 to about 10.
viii. forming a crosslinked network of the polymer and the primary crosslinker;
ix. admixing an active material to the reactant mixture; and
x. encapsulating the active material with the polymer to form a polymer encapsulated material; and
xi. an optional step of adding a secondary crosslinker to the reacted polymer encapsulated material thereby modifying the capsule surface
xii. an optional step of adding a polymer selected from the groups consisting of an amine-containing polymer, an amine-generating polymer and mixtures thereof , to the encapsulated material to provide a multi-shell morphology around the encapsulated material.

The active material may be selected from the group consisting of fragrances, flavoring agents, fungicide, brighteners, antistatic agents, wrinkle control agents, fabric softener actives, hard surface cleaning actives, skin and/or hair conditioning agents, antimicrobial actives, UV protection agents, insect repellants, animal/vermin repellants, flame retardants and mixtures thereof.

The active material may be a fragrance.

The catalyst may be selected from the group consisting of hydrochloric (HCl)acid, sulfuric acid, phosphoric acid, para-toluenesulfonic acid (pTSA), acetic acid, glycolic acid, lactic acid, benzoic acid, citric acid, maleic acid, ammonium chloride, aluminum nitrate, aluminum sulfate, magnesium bromide, magnesium chloride, magnesium nitrate, zinc bromide, zinc iodide, zirconyl nitrate and mixtures thereof.

The crosslinker may be selected from the group consisting of aminoplasts, aldehydes, dialdehydes, epoxy, active oxygen, poly-substituted carboxylic acids and derivatives thereof, diketones, halide-substituted, sulfonyl chloride-based organics, inorganic crosslinkers; organic crosslinkers capable of forming azo, azoxy and hydrazo bonds; lactones, lactams, thionyl chloride, phosgene, tannin/tannic acid, polyphenols, free radical crosslinkers and mixtures thereof.

The crosslinker may be an aldehyde selected from the group consisting of formaldehyde, acetaldehyde and mixtures thereof.

The crosslinker may be glutaraldehyde.

The crosslinker may be an active oxygen crosslinker selected from the group consisting of ozone, OH radicals and mixtures thereof.

The crosslinker may be a poly-substituted carboxylic acids selected from the group consisting of acid chlorides, anyhydrides, isocyanates and mixtures thereof.

The crosslinker in one embodiment is a calcium ion (Ca²⁺).

The crosslinker in one embodiment is a free radical selected from the group consisting of benzoyl peroxide, sodium persulfate, azoisobutylnitrile (AIBN) and mixtures thereof.

The polymeric material may undergo crosslinking by radiation.

The polymer may be an acrylamide-acrylic copolymer having a molecular weight in the range of from about 500 to about 5,000,000; and the weight ratio of acrylic acid monomeric units:acrylamide monomeric units is from about 30:1 to about 1:30.

The weight ratio of primary crosslinker:acrylamide-acrylic acid copolymer is in one embodiment in the range of from about 30:1 to about 1:30.

The mole ratio of acrylic acid monomeric units:acrylamide monomeric units is in one embodiment from about 7:3 to about 3:7.

The weight ratio of primary crosslinker:acrylamide-acrylic acid copolymer may be in the range of from about 14:1 to about 1:14.

The weight ratio of primary crosslinker:acrylamide-acrylic acid copolymer may be in the range of from about 7:1 to about 1:7.

The acrylamide-acrylic acid copolymer has a molecular weight which may be in the range of from about 10,000 to about 100,000.

The polymer may be from the group consisting of an amine-containing polymer, an amine-generating polymer and mixtures thereof, wherein the polymer has a molecular weight in the range of from about 5,000 to about 1,000,000.

The amine-containing polymer may be from the group consisting of polyvinyl amines, polyvinyl formamides, polyallyl amines, gelatin, zein, albumen, polysaccharides and mixtures thereof.

The amine-generating polymer is in one embodiment vinyl formamide.

The amine-generating polymer may further comprise functional groups selected from the group consisting of imines, amides, enamines, N-nitroso, urea, urethane, ion-paired amine salts, oximes, azo, azoxy, hydrazo and mixtures thereof.

The weight ratio of primary crosslinker: amine-containing polymer may be in the range of from about 30:1 to about 1:30.

The weight ratio of primary crosslinker: amine-containing polymer may be in the range of from about 14:1 to about 1:14.

The weight ratio of primary crosslinker: amine-containing polymer may be in the range of from about 7:1 to about 1:7.

It will be appreciated that optional features of embodiments of the invention can be used in relation to other embodiments of the invention. The optional features may be used in any number, and in any combination. This includes, but is not limited to, any combination (in any number) of the dependent claims with any of the independent claims.

### Brief Description of the Drawings

Figure 1 is a graph depicting the enhanced fragrance levels of clothes washed with fabric conditioner containing synthetic amine containing polymer capsules as compared to fabric conditioner with neat fragrance and a fabric conditioner containing standard capsules.
Figure 2 is a graph depicting the enhanced fragrance levels of clothes washed with fabric conditioner containing capsules formed in the presence of an acid catalyst as compared to fabric conditioner with neat fragrance and a fabric conditioner containing standard capsules.
Figure 3 is a graph depicting the enhanced fragrance levels of clothes washed with fabric conditioner containing capsules formed in the presence of a metal salt catalyst as compared to fabric conditioner with neat fragrance and a fabric conditioner containing standard capsules.
Figure 4 is a graph depicting the enhanced fragrance levels of clothes washed with fabric conditioner containing capsules formed in the presence of an acid and a metal salt catalyst as compared to fabric conditioner with neat fragrance and a fabric conditioner containing standard capsules.

### Detailed Description of the Invention

The active material suitable for use in the present invention can be a wide variety of materials in which one would want to deliver in a controlled-release manner onto the surfaces being treated with the present compositions or into the environment surrounding the surfaces. Non-limiting examples of active materials include perfumes, flavoring agents, fungicide, brighteners, antistatic agents, wrinkle control agents, fabric softener actives, hard surface cleaning actives, skin and/or hair conditioning agents, antimicrobial actives, UV protection agents, insect repellants, animal/vermin repellants, flame retardants, and the like.

In a preferred embodiment, the active material is a fragrance, in which case the microcapsules containing fragrance provide a controlled-release scent onto the surface being treated or into the environment surrounding the surface. In this case, the fragrance can be comprised of a number of fragrance raw materials known in the art, such as essential oils, botanical extracts, synthetic fragrance materials, and the like.

In general, the active material is contained in the microcapsule at a level of from about 1% to about 99%, preferably from about 10% to about 95%, and more preferably from about 30% to about 90%, by weight of the total microcapsule. The weight of the total microcapsule includes the weight of the shell of the microcapsule plus the weight of the material inside the microcapsule. An encapsulated malodour counteractant composition, may be contained in mirocapsules at the same range of levels. Of course if both active material and an malodour counteractant composition are contained in the same microcapsule, the total percentage of these components will never exceed 100%.

Microcapsules containing an active material, preferably perfume, suitable for use in the present compositions are described in detail in, e.g., U.S. Pat. Nos. 3,888,689; 4,520,142; 5,126,061; and 5,591,146.

The present compositions optionally, but preferably, further comprise one or more malodour counteractant composition at a level of from about 0.001% to about 99.99%, preferably from about 0.002% to about 99.9%, and more preferably from about 0.005% to about 99%, by weight of the malodour counteractant composition. When the compositions are aqueous liquid compositions (especially non-aerosol compositions) to be sprayed onto surfaces, such as fabrics, the compositions will preferably comprise less than about 20%, preferably less than about 10%, more preferably less than about 5%, by weight of the composition, of malodour counteractant composition. The malodour counteractant composition serves to reduce or remove malodor from the surfaces or objects being treated with the present compositions. The malodour counteractant composition is preferably selected from the group consisting of: uncomplexed cyclodextrin; odor blockers; reactive aldehydes; flavanoids; zeolites; activated carbon; and mixtures thereof. Compositions herein that comprise odor control agents can be used in methods to reduce or remove malodor from surfaces treated with the compositions.

Specific examples of malodour counteractant composition components useful in the aminoplast microencapsulates used in the composition and process of our invention are as follows:
Malodour Counteractant Component Group I:
   1-cyclohexylethan-1-yl butyrate;
   1-cyclohexylethan-1-yl acetate;
   1-cyclohexylethan-l-ol;
   1-(4'-methylethyl)cyclohexylethan-1-yl propionate; and
   2'-hydroxy-1'-ethyl(2-phenoxy)acetate
   each of which compound is marketed under the trademark VEILEX by International Flavors & Fragrances Inc., New York, N.Y., U.S.A.
Malodour Counteractant Component Group II, as disclosed in U.S. Patent 6,379,658:
   β-naphthyl methyl ether;
   β-naphthyl ketone;
   benzyl acetone;
   mixture of hexahydro-4,7-methanoinden-5-yl propionate and hexahydro-4,7-methanoinden-6-yl propionate;
   4-(2,6,6-trimethyl-2-cyclohexen-1-yl)-3-methyl-3-buten-2-one;
   3,7-dimethyl-2,6-nonadien-1-nitrile;
   dodecahydro-3a,6,6,9a-tetramethylnaphtho(2,1-b)furan;
   ethylene glycol cyclic ester of n-dodecanedioic acid;
   1-cyclohexadecen-6-one;
   1-cycloheptadecen-10-one; and
   corn mint oil.

The fragrances suitable for use in this invention include without limitation, any combination of fragrance, essential oil, plant extract or mixture thereof that is compatible with, and capable of being encapsulated by a polymer.

Many types of fragrances can be employed in the present invention, the only limitation being the compatibility and ability to be encapsulated by the polymer being employed, and compatibility with the encapsulation process used. Suitable fragrances include but are not limited to fruits such as almond, apple, cherry, grape, pear, pineapple, orange, strawberry, raspberry; musk, flower scents such as lavender-like, rose-like, iris-like, and carnation-like. Other pleasant scents include herbal scents such as rosemary, thyme, and sage; and woodland scents derived from pine, spruce and other forest smells. Fragrances may also be derived from various oils, such as essential oils, or from plant materials such as peppermint, spearmint and the like. Other familiar and popular smells can also be employed such as baby powder, popcorn, pizza, cotton candy and the like in the present invention.

A list of suitable fragrances is provided in U.S. Patents 4,534,891, 5,112,688 and 5,145,842. Another source of suitable fragrances is found in Perfumes Cosmetics and Soaps, Second Edition, edited by W. A. Poucher, 1959. Among the fragrances provided in this treatise are acacia, cassie, chypre, cylamen, fern, gardenia, hawthorn, heliotrope, honeysuckle, hyacinth, jasmine, lilac, lily, magnolia, mimosa, narcissus, freshly-cut hay, orange blossom, orchids, reseda, sweet pea, trefle, tuberose, vanilla, violet, wallflower, and the like.

As disclosed in commonly assigned U.S. Application No. 10/983,142, the logP of many perfume ingredients has been reported, for example, the Ponoma92 database, available from Daylight Chemical Information Systems, Inc. (Daylight CIS) Irvine, California. The values are most conveniently calculated using ClogP program also available from Daylight CIS. The program also lists experimentally determined logP values when available from the Pomona database. The calculated logP (ClogP) is normally determined by the fragment approach on Hansch and Leo (A. Leo, in Comprehensive Medicinal Chemistry, Vol. 4, C. Hansch, P. G. Sammens, J.B. Taylor and C.A. Ransden, Editiors, p. 295 Pergamon Press, 1990). This approach is based upon the chemical structure of the fragrance ingredient and takes into account the numbers and types of atoms, the atom connectivity and chemical bonding. The ClogP values which are most reliable and widely used estimates for this physiochemical property can be used instead of the experimental LogP values useful in the present invention. Further information regarding ClogP and logP values can be found in U.S. Patent 5,500,138.

Fragrance materials with lower logP or ClogP (these terms will be used interchangeably from this point forward) exhibit higher aqueous solubility. Thus, when these materials are in the core of a capsule which is placed in an aqueous system, they will have a greater tendency to diffuse into the base if the shell wall is permeable to the fragrance materials. Without wishing to be bound by theory, it is believed that normally the mechanism of leaching from the capsule proceeds in three steps in an aqueous base. First, fragrance dissolves into the water that hydrates the shell wall. Second, the dissolved fragrance diffuses through the shell wall into the bulk water phase. Third, the fragrance in the water phase is absorbed by the hydrophobic portions of the surfactant dispersed in the base, thus allowing leaching to continue.

This situation may be improved by one embodiment of the present invention which involves the use of a vast preponderance of high ClogP fragrance materials. In this embodiment of the invention greater than about 60 weight percent of the fragrance materials have a ClogP of greater than 3.3. In another highly preferred embodiment of the invention more than 80 weight percent of the fragrances have a ClogP value of greater than about 4.0. Use of fragrance materials as described previously reduces the diffusion of fragrance through the capsule wall and into the base under specific time, temperature, and concentration conditions.

The following fragrance ingredients provided in Table I are among those suitable for inclusion within the capsule of the present invention:

**TABLE 1**

| **PERFUME INGREDIENTS** | **CLOGP** |
|---|---|
| Allyl cyclohexane propionate | 3.935 |
| Ambrettolide | 6.261 |
| Amyl benzoate | 3.417 |
| Amyl cinnamate | 3.771 |
| Amyl cinnamic aldehyde | 4.324 |
| Amyl cinnamic aldehyde dimethyl acetal | 4.033 |
| Iso-amyl salicylate | 4.601 |
| Aurantiol (Trade name for Hydroxycitronellalmethylanthranilate) | 4.216 |
| Benzyl salicylate | 4.383 |
| para-tert-Butyl cyclohexyl acetate | 4.019 |
| Iso butyl quinoline | 4.193 |
| beta-Caryophyllene | 6.333 |
| Cadinene | 7.346 |
| Cedrol | 4.530 |
| Cedryl acetate | 5.436 |
| Cedryl formate | 5.070 |
| Cinnamyl cinnamate | 5.480 |
| Cyclohexyl salicylate | 5.265 |
| Cyclamen aldehyde | 3.680 |
| Diphenyl methane | 4.059 |
| Diphenyl oxide | 4.240 |
| Dodecalactone | 4.359 |
| Iso E Super (Trade name for 1-(1,2,3,4,5,6,7,8- Octahydro-2,3,8,8-tetramethyl-2-naphthalenyl)-ethanone) | 3.455 |
| Ethylene brassylate | 4.554 |
| Ethyl undecylenate | 4.888 |
| Exaltolide (Trade name for 15-Hydroxyentadecanloic acid, lactone) | 5.346 |
| Galaxolide (Trade name for 1,3,4,6,7,8-Hexahydro- 4,6,6,7,8,8-hexamethylcyclopenta-gamma-2-benzopyran) | 5.482 |
| Geranyl anthranilate | 4.216 |
| Geranyl phenyl acetate | 5.233 |
| Hexadecanolide | 6.805 |
| Hexenyl salicylate | 4.716 |
| Hexyl cinnamic aldehyde | 5.473 |
| Hexyl salicylate | 5.260 |
| Alpha-Irone | 3.820 |
| Lilial (Trade name for para-tertiary-Butyl-alpha-methyl hydrocinnamic aldehyde) | 3.858 |
| Linalyl benzoate | 5.233 |
| Methyl dihydrojasmone | 4.843 |
| Gamma-n-Methyl ionone | 4.309 |
| Musk indanone | 5.458 |
| Musk tibetine | 3.831 |
| Oxahexadecanolide-10 | 4.336 |
| Oxahexadecanolide-11 | 4.336 |
| Patchouli alcohol | 4.530 |
| Phantolide (Trade name for 5-Acetyl-1,1,2,3,3,6-hexamethyl indan) | 5.977 |
| Phenyl ethyl benzoate | 4.058 |
| Phenylethylphenylacetate | 3.767 |
| Phenyl heptanol | 3.478 |
| Alpha-Santalol | 3.800 |
| Thibetolide (Trade name for 15-Hydroxypentadecanoic acid, lactone) | 6.246 |
| Delta-Undecalactone | 3.830 |
| Gamma-Undecalactone | 4.140 |
| Vetiveryl acetate | 4.882 |
| Ylangene | 6.268 |

The higher ClogP materials are preferred, meaning that those materials with a ClogP value of 4.5 are preferred over those fragrance materials with a ClogP of 4; and those materials are preferred over the fragrance materials with a ClogP of 3.3.

The fragrance formulation of the present invention should have at least about 60 weight percent of materials with ClogP greater than 3.3, preferably greater than about 80 and more preferably greater than about 90 weight percent of materials with ClogP greater than 4.

Those with skill in the art appreciate that fragrance formulations are frequently complex mixtures of many fragrance ingredients. A perfumer commonly has several thousand fragrance chemicals to work from. Those with skill in the art appreciate that the present invention may contain a single ingredient, but it is much more likely that the present invention will comprise at least eight or more fragrance chemicals, more likely to contain twelve or more and often twenty or more fragrance chemicals. The present invention also contemplates the use of complex fragrance formulations containing fifty or more fragrance chemicals, seventy five or more or even a hundred or more fragrance chemicals in a fragrance formulation.

Preferred fragrance materials will have both high ClogP and high vapor pressure. Among those having these properties are:
Para cymene, Caphene, Mandarinal Firm, Vivaldie, Terpinene, Verdox, Fenchyl acetate, Cyclohexyl isovalerate, Manzanate, Myrcene, Herbavert, Isobutyl isobutyrate, Tetrahydrocitral, Ocimene and Caryophyllene.

As described herein, the present invention is well suited for use in a variety of well-known consumer products such as laundry detergent and fabric softeners, liquid dish detergents, automatic dish detergents, as well as hair shampoos and conditioners. These products employ surfactant and emulsifying systems that are well known. For example, fabric softener systems are described in U.S. Patents 6,335,315, 5,674,832, 5,759,990, 5,877,145, 5,574,179; 5,562,849, 5,545,350, 5,545,340, 5,411,671, 5,403,499, 5,288,417, and 4,767,547, 4,424,134. Liquid dish detergents are described in U.S. Patents 6,069,122 and 5,990,065; automatic dish detergent products are described in U.S. Patents 6,020,294, 6,017,871, 5,968,881, 5,962,386, 5,939,373, 5,914,307, 5,902,781, 5,705,464, 5,703,034, 5,703,030, 5,679,630, 5,597,936, 5,581,005, 5,559,261, 4,515,705, 5,169,552, and 4,714,562. Liquid laundry detergents which can use the present invention include those systems described in U.S. Patents 5,929,022, 5,916,862, 5,731,278, 5,565,145, 5,470,507, 5,466,802, 5,460,752, 5,458,810, 5,458,809, 5,288,431,5,194,639, 4,968,451, 4,597,898, 4,561,998, 4,550,862, 4,537,707, 4,537,706, 4,515,705, 4,446,042, and 4,318,818. Shampoo and conditioners that can employ the present invention include those described in U.S. Patents 6,162,423, 5,968,286, 5,935,561, 5,932,203, 5,837,661, 5,776,443, 5,756,436, 5,661,118, 5,618,523, 5,275,755, 5,085,857, 4,673,568, 4,387,090 and 4,705,681. All of the above mentioned U.S. Patents.

In addition to the fragrance materials that are to be encapsulated in the present invention, the present invention also contemplates the incorporation of solvent materials. The solvent materials are hydrophobic materials that are miscible in the fragrance materials used in the present invention. Suitable solvents are those having reasonable affinity for the fragrance chemicals and a ClogP greater than 3.3, preferably greater than 8 and most preferably greater that 10. Suitable materials include, but are not limited to triglyceride oil, mono and diglycerides, mineral oil, silicone oil, diethyl phthalate, polyalpa olefins, castor oil and isopropyl myristate. In a preferred embodiment the solvent materials are combined with fragrance materials that have high ClogP values as set forth above. It should be noted that selecting a solvent and fragrance with high affinity for each other will result in the most pronounced improvement in stability. Appropriate solvents may be selected from the following non-limiting list:
- Mono-, di- and tri-esters, and mixtures thereof, of fatty acids and glycerine. The fatty acid chain can range from C4-C26. Also, the fatty acid chain can have any level of unsaturation. For instance capric/caprylic triglyceride known as Neobee M5 (Stepan Corporation). Other suitable examples are the Capmul series by Abitec Corporation. For instance, Capmul MCM.
- Isopropyl myristate
- Fatty acid esters of polyglycerol oligomers:
   R2CO-[OCH₂-CH(OCOR1)-CH₂O-]n, where R1 and R2 can be H or C4-26 aliphatic chains, or mixtures thereof, and n ranges between 2 - 50, preferably 2-30.
- Nonionic fatty alcohol alkoxylates like the Neodol surfactants by BASF, the Dobanol surfactants by Shell Corporation or the BioSoft surfactants by Stepan. The alkoxy group being ethoxy, propoxy, butoxy, or mixtures thereof. In addition, these surfactants can be end-capped with methyl groups in order to increase their hydrophobicity.
- Di- and tri-fatty acid chain containing nonionic, anionic and cationic surfactants, and mixtures thereof.
- Fatty acid esters of polyethylene glycol, polypropylene glycol, and polybutylene glycol, or mixtures thereof.
- Polyalphaolefins such as the ExxonMobil PureSym™ PAO line
- Esters such as the ExxonMobil PureSyn™ Esters
- Mineral oil
- Silicone oils such polydimethyl siloxane and polydimethylcyclosiloxane
- Diethyl phthalate
- Di-isodecyl adipate

The level of solvent in the core of the encapsulated fragrance material should be greater than about 30 weight percent, preferably greater than about 50 weight percent and most preferably greater than about 75 weight percent. In addition to the solvent it is preferred that higher ClogP fragrance materials are employed. It is preferred that greater than about 25 weight percent, preferably greater than 30 and more preferably greater than about 40 weight percent of the fragrance chemicals have ClogP values of greater than about 2.5, preferably greater than about 3 and most preferably greater than about 3.5. Those with skill in the art will appreciate that many formulations can be created employing various solvents and fragrance chemicals. The use of high ClogP fragrance chemicals will require a lower level of hydrophobic solvent than fragrance chemicals with lower ClogP to achieve similar stability. As those with skill in the art will appreciate, in a highly preferred embodiment high ClogP fragrance chemicals and hydrophobic solvents comprise greater than about 80, preferably more than about 90 and most preferably greater than 99 weight percent of the fragrance composition.

It has also been found that the addition of hydrophobic polymers to the core can also improve stability by slowing diffusion of the fragrance from the core. The level of polymer is normally less than 80% of the core by weight, preferably less than 50%, and most preferably less than 20%. The basic requirement for the polymer is that it be miscible or compatible with the other components of the core, namely the fragrance and other solvent. Preferably, the polymer also thickens or gels the core, thus further reducing diffusion. Polymers may be selected from the non-limiting group below:
- Copolymers of ethylene. Copolymers of ethylene and vinyl acetate (Elvax polymers by DOW Corporation). Copolymers of ethylene and vinyl alcohol (EVAL polymers by Kuraray). Ethylene/Acrylic elastomers such as Vamac polymers by Dupont).
- Poly vinyl polymers, such as poly vinyl acetate.
- Alkyl-substituted cellulose, such as ethyl cellulose (Ethocel made by DOW Corporation), hydroxypropyl celluloses (Klucel polymers by Hercules)
- Uncharged polyacrylates. Examples being (i) Amphomer, Demacryl LT and Dermacryl 79, made by National Starch and Chemical Company, (ii) the Amerhold polymers by Amerchol Corporation, and (iii) Acudyne 258 by ISP Corporation.
- Copolymers of acrylic or methacrylic acid and fatty esters of acrylic or methacrylic acid. These are side-chain crystallizing. Typical polymers of this type are those listed in U.S. Patents 4,830,855, 5,665,822, 5,783,302, 6,255,367 and 6,492,462. Examples of such polymers are the Intelimer Polymers, made by Landec Corporation.
- Polypropylene oxide.
- Polybutylene oxide of poly(tetra hydrofuran).
- Polyethylene terephthalate.
- Alkyl esters of poly(methyl vinyl ether) - maleic anhydride copolymers, such as the Gantrez copolymers and Omnirez 2000 by ISP Corporation.
- Carboxylic acid esters of polyamines. Examples of this are ester-terminated polyamide (ETPA) made by Arizona Chemical Company.
- Poly vinyl pyrrolidone (Luviskol series of BASF).
- Block copolymers of ethylene oxide, propylene oxide and/or butylenes oxide. These are known as the Pluronic and Synperonic polymers/dispersants by BASF.
- Another class of polymers include polyethylene oxide-co-propyleneoxide-co-butylene oxide polymers of any ethylene oxide/propylene oxide / butylene oxide ratio with cationic groups resulting in a net theoretical positive charge or equal to zero (amphoteric). The general structure is: 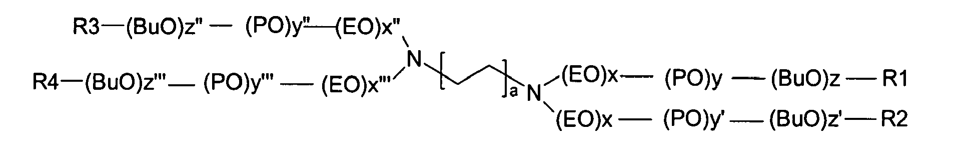
where R1, 2, 3, 4 is H or any alkyl of fatty alkyl chain group. The value for 'a' can range from 1-100. Examples of such polymers are the commercially known as Tetronics by BASF Corporation.

We have also discovered that when capsules having cores containing a very large proportion of solvents with the appropriate ClogP values and/or with the high ClogP fragrance chemicals described above the encapsulated materials are actually capable of absorbing fragrance chemicals from surfactant-containing product bases. As is well appreciated by those with skill in the art, products such as, but not limited to fabric softeners, laundry detergents, bleaching products, shampoos and hair conditioners contain in their base formulas functional materials such as surfactants, emulsifying agents, detergent builders, whiteners, and the like along with fragrance chemicals. These products often aggressively absorb fragrance ingredients, most often due to the partially hydrophobic surfactant.

Most consumer products are made using an aqueous base, although some products use glycols, polyhydric alcohols, alcohols, or silicone oils as the dominant solvent or carrier. Absorption from these bases is also possible if the core is properly designed and used at the appropriate level in the base. Examples of these products include many deodorants and antiperspirants.

In the product base the fragrance is used to provide the consumer with a pleasurable fragrance during and after using the product or to mask unpleasant odors from some of the functional ingredients used in the product. As stated above, one long standing problem with the use of fragrance in product bases is the loss of the fragrance before the optimal time for fragrance delivery. We have discovered that with the proper selection of solvent and/or fragrance chemicals in the capsule core, the capsule will successfully compete for the fragrance chemicals present in the aqueous product base during storage. Eventually the core absorbs a significant quantity of fragrance, and finally an equilibrium level of fragrance is established in the core which is specific to the starting core composition and concentration in the base, type and concentration of the fragrance materials in the base, base composition, and conditions of storage. This ability to load the capsule core with fragrance material from the product base, particularly those product bases that contain a high concentration of surfactant proves that with judicious selection of core composition good fragrance stability within the core can be achieved.

As used herein stability of the products is measured at room temperature or above over a period of at least a week. More preferably the capsules of the present invention are allowed to be stored at room temperature for more than about two weeks and preferably more than about a month.

In another embodiment of the invention a sacrificial solvent is initially placed with the capsule. A sacrificial solvent is a solvent having a low ClogP value of from about 1 to about 3, preferably from about 1.25 to about 2.5, and most preferably from about 1.5 to about 2. If the ClogP of the sacrificial solvent is too low, the sacrificial solvents will be lost in the manufacture of the capsule materials. Suitable sacrificial solvents include benzyl acetate, and octanol.

Preferably more than 30 and more than 40 weight percent of the sacrificial solvent will migrate from the capsules to the environment, thereby allowing the capsules to increase the level of high ClogP fragrance material inside the capsule by more than 10 weight percent, preferably more than 20 and most preferably more than 30 weight percent over the original weight of ClogP materials above 3.3 originally found inside the capsule.

An important advantage of the migration technology is that capsules containing sacrificial solvent can be prepared in large quantities, and placed in various fragrance environments. This means that through the proper selection of fragrance materials, capsules and sacrificial solvent, an encapsulated fragrance materials can be prepared without having to encapsulate each specific custom fragrance.

The invention in its various embodiments provides a capsule core composition that is able to retain a significant amount of fragrance within the capsule core and to deliver the higher level of fragrance contained therein at the desired time. We have discovered that the capsule products of the present invention under specified times of time, temperature, and concentration in various product bases retain more than about 10 weight percent, preferably more than 30 and most preferably more than 70 weight percent of the fragrance materials originally encapsulated.

Fragrance retention within the capsule may be measured directly after storage at a desired temperature and time periods such as six weeks, two months, three months or more. The preferred manner is to measure total headspace of the product at the specified time and to compare the results to the headspace of a control product made to represent 0% retention via direct addition of the total amount of fragrance present. Alternatively, the product base may be performance tested after the storage period and the performance compared to the fresh product, either analytically or by sensory evaluation. This more indirect measurement often involves either measuring the fragrance headspace over a substrate used with the product, or odor evaluation of the same substrate.

As used herein olfactory effective amount is understood to mean the amount of compound in perfume compositions the individual component will contribute to its particular olfactory characteristics, but the olfactory effect of the fragrance composition will be the sum of the effects of each of the fragrance ingredients. Thus the compounds of the invention can be used to alter the aroma characteristics of the perfume composition by modifying the olfactory reaction contributed by another ingredient in the composition. The amount will vary depending on many factors including other ingredients, their relative amounts and the effect that is desired.

The level of fragrance in the cationic polymer coated encapsulated fragrance varies from about 5 to about 95 weight percent, preferably from about 40 to about 95 and most preferably from about 50 to about 90 weight percent on a dry basis. In addition to the fragrance other agents can be used in conjunction with the fragrance and are understood to be included.

As noted above, the fragrance may also be combined with a variety of solvents which serve to increase the compatibility of the various materials, increase the overall hydrophobicity of the blend, influence the vapor pressure of the materials, or serve to structure the blend. Solvents performing these functions are well known in the art and include mineral oils, triglyceride oils, silicone oils, fats, waxes, fatty alcohols, diisodecyl adipate, and diethyl phthalate among others.

A common feature of many encapsulation processes is that they require the fragrance material to be encapsulated to be dispersed in aqueous solutions of polymers, pre-condensates, surfactants, and the like prior to formation of the capsule walls. Therefore, materials having low solubility in water, such as highly hydrophobic materials are preferred, as they will tend to remain in the dispersed perfume phase and partition only slightly into the aqueous solution. Fragrance materials with Clog P values greater than 1, preferably greater than 3, and most preferably greater than 5 will thus result in micro-capsules that contain cores most similar to the original composition, and will have less possibility of reacting with materials that form the capsule shell.

One object of the present invention is to deposit capsules containing fragrance cores on desired substrates such as cloth, hair, and skin during washing and rinsing processes. Further, it is desired that, once deposited, the capsules release the encapsulated fragrance either by diffusion through the capsule wall, via small cracks or imperfections in the capsule wall caused by drying, physical, or mechanical means, or by large-scale rupture of the capsule wall. In each of these cases, the volatility of the encapsulated perfume materials is critical to both the speed and duration of release, which in turn control consumer perception. Thus, fragrance chemicals which have higher volatility as evidenced by normal boiling points of less than 250°C, preferably less than about 225°C are preferred in cases where quick release and impact of fragrance is desired. Conversely, fragrance chemicals that have lower volatility (boiling points greater than 225°C) are preferred when a longer duration of aroma is desired. Of course, fragrance chemicals having varying volatility may be combined in any proportions to achieve the desired speed and duration of perception.

In order to provide the highest fragrance impact from the fragrance encapsulated capsules deposited on the various substrates referenced above, it is preferred that materials with a high odor-activity be used. Materials with high odor-activity can be detected by sensory receptors at low concentrations in air, thus providing high fragrance perception from low levels of deposited capsules. This property must be balanced with the volatility as described above. Some of the principles mentioned above are disclosed in U.S. Patent No. 5,112,688.

Further, it is clear that materials other than fragrances may be employed in the system described here. Examples of other materials which may be usefully deposited from rinse-off products using the invention include sunscreens, softening agents, insect repellents, and fabric conditioners, among others.

Encapsulation of active materials such as fragrances is known in the art, see for example U.S. Patent Nos. 2,800,457, 3,870,542, 3,516,941, 3,415,758, 3,041,288, 5,112,688, 6,329,057, and 6,261,483. Another discussion of fragrance encapsulation is found in the Kirk-Othmer Encyclopedia.

Preferred encapsulating polymers include those formed from melamine-formaldehyde or urea-formaldehyde condensates, as well as similar types of aminoplasts. Additionally, capsules made via the simple or complex coacervation of gelatin are also preferred for use with the coating. Capsules having shell walls comprised of polyurethane, polyamide, polyolefin, polysaccaharide, protein, silicone, lipid, modified cellulose, gums, polyacrylate, polystyrene, and polyesters or combinations of these materials are also functional.

A representative process used for aminoplast encapsulation is disclosed in U.S. Patent No. 3,516,941 though it is recognized that many variations with regard to materials and process steps are possible. A representative process used for gelatin encapsulation is disclosed in U.S. Patent No, 2,800,457 though it is recognized that many variations with regard to materials and process steps are possible. Both of these processes are discussed in the context of fragrance encapsulation for use in consumer products in U.S. Patent Nos. 4,145,184 and 5,112,688 respectively.

Well known materials such as solvents, surfactants, emulsifiers, and the like can be used in addition to the polymers described above to encapsulate the active materials such as fragrance without departing from the scope of the present invention. It is understood that the term encapsulated is meant to mean that the fragrance material is substantially covered in its entirety. Encapsulation can provide pore vacancies or interstitial openings depending on the encapsulation techniques employed. More preferably the entire fragrance material portion of the present invention is encapsulated.

Fragrance capsules known in the art consists of a core of various ratios of fragrance and a diluent, a wall or shell comprising a three-dimensional cross-linked network of an aminoplast resin, more specifically a substituted or un-substituted acrylic acid polymer or co-polymer cross-linked with a urea-formaldehyde pre-condensate or a melamine-formaldehyde pre-condensate.

Microcapsule formation using mechanisms similar to the foregoing mechanism, using (i) melamine-formaldehyde or urea-formaldehyde pre-condensates and (ii) polymers containing substituted vinyl monomeric units having proton-donating functional group moieties, e.g., sulfonic acid groups or carboxylic acid anhydride groups, bonded thereto is disclosed in U.S. Patent 4,406,816 (2-acrylamido-2-methyl-propane sulfonic acid groups), UK published Patent Application GB 2,062,570 A (styrene sulfonic acid groups) and UK published Patent Application GB 2,006,709 A (carboxylic acid anhydride groups).

When substituted or un-substituted acrylic acid co-polymers are employed in the practice of our invention, in the case of using a co-polymer having two different monomeric units, e.g., acrylamide monomeric units and acrylic acid monomeric units, the mole ratio of the first monomeric unit to the second monomeric unit is in the range of from about 1:9 to about 9:1, preferably from about 3:7 to about 7:3. In the case of using a co-polymer having three different monomeric units, e.g., ethyl methacrylate, acrylic acid and acrylamide, the mole ratio of the first monomeric unit to the second monomeric unit to the third monomeric unit is in the range of 1:1:8 to about 8:8:1, preferably from about 3:3:7 to about 7:7:3.

As disclosed in copending Application for U.S. Letters Patent Serial Number 10/823,492 filed on April 13, 2004.

The molecular weight range of the substituted or un-substituted acrylic acid polymers or co-polymers useful in the practice of our invention is from about 500 to about 5,000,000, preferably from about 10,000 to about 100,000. The weight ratio of acrylic acid monomeric units:acrylamide monomeric units may be from about 30:1 to about 1:30. Also, the weight ratio of primary crosslinker:acrylamide-acrylic acid copolymer is in the range of from 30:1 to 1:30.

The substituted or un-substituted acrylic acid polymers or co-polymers useful in the practice of our invention may be branched, linear, star-shaped, dendritic-shaped or may be a block polymer or copolymer, or blends of any of the aforementioned polymers or copolymers.

Such substituted or un-substituted acrylic acid polymers or co-polymers may be prepared according to any processes known to those skilled in the art, for example, U.S. Patent 6,545,084.

The urea-formaldehyde and melamine-formaldehyde pre-condensate microcapsule shell wall precursors are prepared by means of reacting urea or melamine with formaldehyde where the mole ratio of melamine or urea to formaldehyde is in the range of from about 10:1 to about 1:6, preferably from about 1:2 to about 1:5. For purposes of practicing our invention, the resulting material has a molecular weight in the range of from 156 to 3000. The resulting material may be used as a cross-linking agent for the aforementioned substituted or un-substituted acrylic acid polymer or copolymer or it may be further reacted with a C₁-C₆ alkanol, e.g., methanol, ethanol, 2-propanol, 3-propanol, 1-butanol , 1-pentanol or 1-hexanol, thereby forming a partial ether where the mole ratio of melamine or urea:formalhyde:alkanol is in the range of 1:(0.1 - 6):(0.1-6). The resulting ether moiety-containing product may by used as a cross-linking agent for the aforementioned substituted or un-substituted acrylic acid polymer or copolymer, or it may be self-condensed to form dimers, trimers and/or tetramers which may also be used as cross-linking agents for the aforementioned substituted or un-substituted acrylic acid polymers or co-polymers. Methods for formation of such melamine-formaldehyde and urea-formaldehyde pre-condensates are set forth in U.S. Patent 3,516,846, U.S. Patent 6,261,483, and Lee et al. J. Microencapsulation, 2002, Vol. 19, No. 5, pp 559-569, "Microencapsulation of fragrant oil via in situ polymerization: effects of pH and melamine-formaldehyde molar ratio". Examples of urea-formaldehyde pre-condensates useful in the practice of our invention are URAC 180 and URAC 186, Cytec Technology Corp. Examples of melamine-formaldehyde pre-condensates useful in the practice of our invention are CYMEL U-60, CYMEL U-64 and CYMEL U-65, Cytec Technology Corp.

In one embodiment of the invention, the range of mole ratios of urea-formaldehyde precondensate or melamine-formaldehyde pre-condensate:substituted or un-substituted acrylic acid polymer or co-polymer is in the range of from about 9:1 to about 1:9, preferably from about 5:1 to about 1:5 and most preferably from about 1:2 to about 2:1.

In one embodiment of the invention, capsules with polymer(s) comprising primary and/or secondary amine reactive groups or mixtures thereof and crosslinkers are provided. The amine polymers can possess primary and/or secondary amine functionalities and can be of either natural or synthetic origin. Amine containing polymers of natural origin are typically proteins such as gelatin and albumen, as well as some polysaccharides. Synthetic amine polymers include various degrees of hydrolyzed polyvinyl formamides, polyvinylamines, polyallyl amines and other synthetic polymers with primary and secondary amine pendants. Examples of suitable amine polymers are the Lupamin series of polyvinyl formamides (available from BASF). The molecular weights of these materials can range from 10,000 to 1,000,000.

The polymers containing primary and/or secondary amines can be used with any of the following comonomers in any combination:
1. Vinyl and acrylic monomers with:
   a. alkyl, aryl and silyl substituents;
   b. OH, COOH, SH, aldehyde, trimonium, sulfonate, NH₂, NHR substiuents;
   c. vinyl pyridine, vinyl pyridine-N-oxide, vinyl pyrrolidon
2. Cationic monomers such as dialkyl dimethylammonium chloride, vinyl imidazolinium halides, methylated vinyl pyridine, cationic acrylamides and guanidine-based monomers
3. N-vinyl formamide
   and any mixtures thereof. The ratio amine monomer/total monomer ranges from 0.01 - 0.99, more preferred from 0.1 - 0.9.

The following represents a general formula for the amine-containing polymer material: 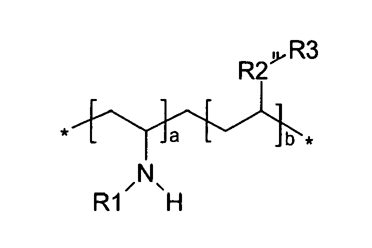 wherein R is a saturated or unsaturated alkane, dialkylsiloxy, dialkyloxy, aryl, alkylated aryl, and that may further contain a cyano, OH, COOH, NH₂, NHR, sulfonate, sulphate, -NH₂, quaternized amines, thiols, aldehyde, alkoxy, pyrrolidone, pyridine, imidazol, imidazolinium halide, guanidine, phosphate, monosaccharide, oligo or polysaccharide.

R1 is H, CH₃, (C=O)H, alkylene, alkylene with unsaturated C-C bonds, CH₂-CROH, (C=O) -NH-R, (C=O) - (CH₂) n-OH, (C=O) -R, (CH₂)n-E, -(CH₂-CH(C=O))n-XR, - (CH₂)n-COOH, - (CH₂)n-NH₂, -CH₂)n-(C=O)NH₂, E is an electrophilic group; wherein a and b are integers or average numbers (real numbers) from about 100-25,000.

R2 can be nonexistent or the functional group selected from the group consisting of -COO-, -(C=O)-, -O-, -S-, -NH-(C=O)-, -NR1-, dialkylsiloxy, dialkyloxy, phenylene, naphthalene, alkyleneoxy. R3 can be the same or selected from the same group as R1.

Additional copolymers with amine monomers are provided having the structure: 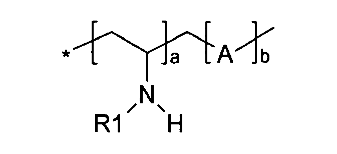 R1 is H, CH₃, (C=O)H, alkylene, alkylene with unsaturated C-C bonds, CH₂-CROH, (C=O) -NH-R, (C=O)-(CH₂)n-OH, (C=O) -R, (CH₂)n-E, -(CH₂-CH (C=O)) n-XR, - (CH₂)n-COOH, - (CH₂)n-NH₂, -CH₂)n- (C=O) NH₂, E is an electrophilic group; wherein a and b are integers or average numbers (real numbers) from about 100-25,000.

The comonomer, represented by A, can contain an amine monomer and a cyclic monomer wherein A can be selected from the group consisting of aminals, hydrolyzed or non-hydrolyzed maleic anhydride, vinyl pyrrolidine, vinyl pyridine, vinyl pyridine-N-oxide, methylated vinyl pyridine, vinyl naphthalene, vinyl naphthalene-sulfonate and mixtures thereof.

When A is an aminal the following general structure can represent the aminal: 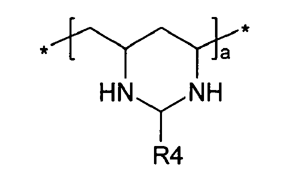 wherein R4 is selected from the group consisting of H, CH₃, (C=O)H, alkylene, alkylene with unsaturated C-C bonds, CH₂-CROH, (C=O) -NH-R, (C=O)-(CH₂)n-OH, (C=O) -R, (CH₂)n-E, -(CH₂-CH(C=O))n-XR, -(CH₂)n-COOH, -(CH₂)n-NH2, -CH₂)n-(C=O)NH₂, E is an electrophilic group; wherein R is a saturated or unsaturated alkane, dialkylsiloxy, dialkyloxy, aryl, alkylated aryl, and that may further contain a cyano, OH, COOH, NH₂, NHR, sulfonate, sulphate, -NH₂, quaternized amines, thiols, aldehyde, alkoxy, pyrrolidone, pyridine, imidazol, imidazolinium halide, guanidine, phosphate, monosaccharide, oligo or polysaccharide.

In addition instead of amine-containing polymers it is possible to utilize amine-generating polymers that can generate primary and secondary amines during the capsule formation process.

The benefits of the preferred embodiment are that these capsules have better leaching stability in certain product bases as compared to standard aminoplast capsules. Additional benefits are that the capsules can deposit better on their own as because (i) they have the potential of being cationically charged at pH of about 8 and less, and/or (ii) the improved adhesion force of unreacted amine functionalities. The capsules also have less interaction with anionic surfactant as the capsule surface is not strongly positively charged. These additional benefits eliminate the need for deposition aids in specific applications.

The crosslinkers can be selected from the group consisting of aminoplasts, aldehydes such as formaldehyde and acetaldehyde, dialdehydes such as glutaraldehyde, epoxy, active oxygen such as ozone and OH radicals, poly-substituted carboxylic acids and derivatives such as acid chlorides, anyhydrides, isocyanates, diketones, halide-substituted, sulfonyl chloride-based organics, inorganic crosslinkers such as Ca²⁺, organics capable of forming azo, azoxy and hydrazo bonds, lactones and lactams, thionyl chloride, phosgene, tannin/tannic acid, polyphenols and mixtures thereof. Furthermore, processes such as free radical and radiation crosslinking can be used according to the present invention. Examples of free radical crosslinkers are benzoyl peroxide, sodium persulfate, azoisobutylnitrile (AIBN) and mixtures thereof.

In a further embodiment a crosslinker can be added to the encapsulated material once the reaction has completed. The additional crosslinker reacts with itself and also with the unreacted groups on the capsule surface.

The encapsulated active material and/or odor controlling agents with amine-containing polymers provides the advantage that the encapsulated materials contain a core and a shell and do not require further coating by a cationic polymer thereby saving processing time and the additional costs incurred in processing.

The microcapsule walls can be composed of an amine-containing polymer and/or amine-generating polymer which may be combined with a comonomers and mixtures thereof, cross-linked with a crosslinker such as but not limited to formaldehyde pre-condensate such as a urea or melamine-formaldehyde pre-condensate. The microcapsule is formed by means of either (a) forming an aqueous dispersion of a non-cured amine-containing polymer or co-polymer by reacting under appropriate pH conditions being from about 2 to about 10, preferably about 3 to about 9 and more preferably about 4 and about 8, a urea-formaldehyde pre-condensate or a melamine-formaldehyde pre-condensate with one or more substituted or un-substituted amine-containing polymer or co-polymer; then adsorbing the resulting non-cured amine-containing polymer shell about the surface of a fragrance-solvent monophasic droplet under homogenization conditions (e.g. using a homogenization apparatus as described in U.S. Patent 6,042,792 and illustrated in Figures 11A and 11B thereof); and then curing the microcapsule shell wall at an elevated temperature, e.g. 50 - 85°C or (b) forming either an amine-containing polymer wall at the surface of the fragrance-solvent monophasic droplet by means of reacting, at the surface of the droplet a urea-formaldehyde pre-condensate or a melamine-formaldehyde pre-condensate with one or more substituted or un-substituted amine-containing polymer or co-polymer, and then curing the microcapsule shell wall at an elevated temperature, e.g. 50 - 85°C, or (c) reacting an amine containing polymer (primary and/or secondary amines) with an aminoplast around an existing capsule wall of any chemistry type as listed on page 20 and 21. In addition multiple shells can be formed by the above process at the required pH and temperature stepwise in any of combination.

Furthermore, in an additional embodiment the amine containing and/or generating polymers described above can be used to provide additional coatings such as multiple shells to any existing capsules known in the art. For example, the amine containing and/or generating polymers of the present invention can be applied in a multi-shell morphology around any existing capsules of any wall chemistry, as disclosed above, so that each of the shells may be comprised of different wall chemistries.

Microcapsule formation using mechanisms similar to the foregoing mechanism, using (i) melamine-formaldehyde or urea-formaldehyde pre-condensates and (ii) polymers containing substituted vinyl monomeric units having proton-donating functional group moieties (e.g. sulfonic acid groups or carboxylic acid anhydride groups) bonded thereto is disclosed in U.S. Patent 4,406,816 (2-acrylamido-2-methyl-propane sulfonic acid groups), UK published Patent Application GB 2,062,570 A (styrene sulfonic acid groups) and UK published Patent Application GB 2,006,709 A (carboxylic acid anhydride groups).
(A) Copolymers containing primary and/or secondary amine. When amine-containing polymers are employed in the practice of the invention, in the case of using a co-polymer having two different monomeric units, e.g. Lupamin 9030 (copolymer of vinyl amine and vinyl formamide), the mole ratio of the first monomeric unit to the second monomeric unit is in the range of from about 0.1:0.9 to about 0.9:0.1, preferably from about 1:9 to about 9:1. In the case of using a co-polymer having three different monomeric units, e.g. a copolymer of vinyl amine, vinyl formamide and acrylic acid, the mole ratio of the reactive monomer (i.e. vinyl amine + acrylic acid) in the total polymer ranging from 0.1-0.9, more preferably from 1-9.
(B) Branched amine containing polymers such as ethylene imines (Lupasol series of BASF) and ethoxylated ethylene imines.
(C) Mixtures of amine containing polymers and other polymers that contain other reactive groups such as COOH, OH, and SH.

The molecular weight range of the substituted or un-substituted amine-containing polymers or co-polymers and mixtures thereof, useful in the practice of our invention is from about 1,000 to about 1,000,000, preferably from about 10,000 to about 500,000. The substituted or un-substituted amine-containing polymers or co-polymers useful in the practice of our invention may be branched, linear, star-shaped, graft, ladder, comb/brush, dendritic-shaped or may be a block polymer or copolymer, or blends of any of the aforementioned polymers or copolymers. Alternatively, these polymers may also possess thermotropic and/or lyotropic liquid crystalline properties.

As disclosed in commonly assigned U.S. Application 10/720,524, particles comprised of fragrance and a variety of polymeric and non-polymeric matrixing materials are also suitable for use. These may be composed of polymers such as polyethylene, fats, waxes, or a variety of other suitable materials. Essentially any capsule, particle, or dispersed droplet may be used that is reasonably stable in the application and release of fragrance at an appropriate time once deposited.

Particle and capsule diameter can vary from about 10 nanometers to about 1000 microns, preferably from about 50 nanometers to about 100 microns and most preferably from about 2 to about 15 microns. The capsule distribution can be narrow, broad, or multi-modal. Each modal of the multi-modal distributions may be composed of different types of capsule chemistries.

Once the fragrance material is encapsulated a cationically charged water-soluble polymer can be applied to the fragrance encapsulated polymer. This water-soluble polymer can also be an amphoteric polymer with a ratio of cationic and anionic functionalities resulting in a net total charge of zero and positive, i.e., cationic. Those skilled in the art would appreciate that the charge of these polymers can be adjusted by changing the pH, depending on the product in which this technology is to be used. Any suitable method for coating the cationically charged materials onto the encapsulated fragrance materials can be used. The nature of suitable cationically charged polymers for assisted capsule delivery to interfaces depends on the compatibility with the capsule wall chemistry since there has to be some association to the capsule wall. This association can be through physical interactions, such as hydrogen bonding, ionic interactions, hydrophobic interactions, electron transfer interactions or, alternatively, the polymer coating could be chemically (covalently) grafted to the capsule or particle surface. Chemical modification of the capsule or particle surface is another way to optimize anchoring of the polymer coating to capsule or particle surface. Furthermore, the capsule and the polymer need to want to go to the desired interface and, therefore, need to be compatible with the chemistry (polarity, for instance) of that interface. Therefore, depending on which capsule chemistry and interface (e.g., cotton, polyester, hair, skin, wool) is used the cationic polymer can be selected from one or more polymers with an overall zero (amphoteric: mixture of cationic and anionic functional groups) or net positive charge, based on the following polymer backbones: polysaccharides, polypeptides, polycarbonates, polyesters, polyolefinic (vinyl, acrylic, acrylamide, poly diene), polyester, polyether, polyurethane, polyoxazoline, polyamine, silicone, polyphosphazine, olyaromatic, poly heterocyclic, or polyionene, with molecular weight (MW) ranging from about 1,000 to about 1000,000,000, preferably from about 5,000 to about 10,000,000. As used herein molecular weight is provided as weight average molecular weight. Optionally, these cationic polymers can be used in combination with nonionic and anionic polymers and surfactants, possibly through coacervate formation.

A more detailed list of cationic polymers that can be used to is provided below:
Polysaccharides include but are not limited to guar, alginates, starch, xanthan, chitosan, cellulose, dextrans, arabic gum, carrageenan, hyaluronates. These polysaccharides can be employed with:
   (a) cationic modification and alkoxy-cationic modifications, such as cationic hydroxyethyl, cationic hydroxy propyl. For example, cationic reagents of choice are 3-chloro-2-hydroxypropyl trimethylammonium chloride or its epoxy version. Another example is graft-copolymers of polyDADMAC on cellulose like in Celquat L-200 (Polyquaternium-4), Polyquaternium-10 and Polyquaternium-24, commercially available from National Starch, Bridgewater, N.J.;
(b) aldehyde, carboxyl, succinate, acetate, alkyl, amide, sulfonate, ethoxy, propoxy, butoxy, and combinations of these functionalities. Any combination of Amylose and Mylopectin and overall molecular weight of the polysaccharide; and
(c) any hydrophobic modification (compared to the polarity of the polysaccharide backbone).

The above modifications described in (a), (b) and (c) can be in any ratio and the degree of functionalization up to complete substitution of all functionalizable groups, and as long as the theoretical net charge of the polymer is zero (mixture of cationic and anionic functional groups) or preferably positive. Furthermore, up to 5 different types of functional groups may be attached to the polysaccharides. Also, polymer graft chains may be differently modified than the backbone. The counterions can be any halide ion or organic counter ion. U.S. Patent Nos. 6,297,203 and U.S. 6,200,554.

Another source of cationic polymers contain protonatable amine groups so that the overall net charge is zero (amphoteric:
mixture of cationic and anionic functional groups) or positive. The pH during use will determine the overall net charge of the polymer. Examples are silk protein, zein, gelatin, keratin, collagen and any polypeptide, such as polylysine.

Further cationic polymers include poly vinyl polymers, with up to 5 different types of monomers, having the monomer generic formula -C(R2)(R1)-CR2R3-. Any co-monomer from the types listed in this specification may also be used. The overall polymer will have a net theoretical positive charge or equal to zero (mixture of cationic and anionic functional groups). Where R1 is any alkanes from C1-C25 or H; the number of double bonds ranges from 0-5. Furthermore, R1 can be an alkoxylated fatty alcohol with any alkoxy carbon-length, number of alkoxy groups and C1-C25 alkyl chain length. R1 can also be a liquid crystalline moiety that can render the polymer thermotropic liquid crystalline properties, or the alkanes selected can result in side-chain melting. In the above formula R2 is H or CH₃; and R3 is -Cl, -NH₂ (i.e., poly vinyl amine or its copolymers with N-vinyl formamide. These are sold under the name Lupamin 9095 by BASF Corporation), -NHR1, -NR1R2, -NR1R2 R6 (where R6 = R1, R2, or -CH2-COOH or its salt), -NH-C(O)-H, -C(O)-NH₂ (amide), -C(O)-N(R2)(R2')(R2"), -OH, styrene sulfonate, pyridine, pyridine-N-oxide, quaternized pyridine, imidazolinium halide, imidazolium halide, imidazol, piperidine, pyrrolidone, alkyl-substituted pyrrolidone, caprolactam or pyridine, phenyl-R4 or naphthalene-R5 where R4 and R5 are R1, R2, R3, sulfonic acid or its alkali salt -COOH, -COO- alkali salt, ethoxy sulphate or any other organic counter ion. Any mixture or these R3 groups may be used. Further suitable cationic polymers containing hydroxy alkyl vinyl amine units, as disclosed in U.S. Patent No 6,057,404.

Another class of materials is polyacrylates, with up to 5 different types of monomers, having the monomer generic formula: -CH(R1)-C(R2)(CO-R3-R4)-. Any co-monomer from the types listed in this specification may also be used. The overall polymer will have a net theoretical positive charge or equal to zero (mixture of cationic and anionic functional groups). In the above formula R1 is any alkane from C1-C25 or H with number of double bonds from 0-5,aromatic moieties, polysiloxane, or mixtures thereof. Furthermore, R1 can be an alkoxylated fatty alcohol with any alkoxy carbon-length, number of alkoxy groups and C1-C25 alkyl chain length. R1 can also be a liquid crystalline moiety that can render the polymer thermotropic liquid crystalline properties, or the alkanes selected can result in side-chain melting. R2 is H or CH₃; R3 is alkyl alcohol Cl-25 or an alkylene oxide with any number of double bonds, or R3 may be absent such that the C=O bond is (via the C-atom) directly connected to R4. R4 can be: -NH2, NHR1, -NR1R2, -NR1R2 R6 (where R6 = R1, R2, or -CH₂-COOH or its salt), -NH-C(O)-, sulfo betaine, betaine, polyethylene oxide, poly(ethyleneoxide/propylene oxide/butylene oxide) grafts with any end group, H, OH, styrene sulfonate, pyridine, quaternized pyridine, alkyl-substituted pyrrolidone or pyridine, pyridine-N-oxide, imidazolinium halide, imidazolium halide, imidazol, piperidine, -OR1, -OH, -COOH alkali salt, sulfonate, ethoxy sulphate, pyrrolidone, caprolactam, phenyl-R4 or naphthalene-R5 where R4 and R5 are R1, R2, R3, sulfonic acid or its alkali salt or organic counter ion. Any mixture or these R3 groups may be used. Also, glyoxylated cationic polyacrylamides can be used. Typical polymers of choice are those containing the cationic monomer dimethylaminoethyl methacrylate (DMAEMA) or methacrylamidopropyl trimethyl ammonium chloride (MAPTAC). DMAEMA can be found in Gafquat and Gaffix VC-713 polymers from ISP. MAPTAC can be found in BASF's Luviquat PQ11 PN and ISP's Gafquat HS100.

Another group of polymers that can be used are those that contain cationic groups in the main chain or backbone. Included in this group are:
(1) polyalkylene imines such as polyethylene imine, commercially available as Lupasol from BASF. Any molecular weight and any degree of crosslinking of this polymer can be used in the present invention;
(2) ionenes having the general formula set forth as - [N(+)R1R2-A1-N(R5)-X-N(R6)-A2-N(+)R3R4-A3] n- 2Z-, as disclosed in U.S. Patent Nos. 4,395,541 and U.S. 4,597,962;
(3) adipic acid/dimethyl amino hydroxypropyl diethylene triamine copolymers, such as Cartaretin F-4 and F-23, commercially available from Sandoz;
(4) polymers of the general formula- [N(CH₃)₂- (CH₂)x-NH-(CO)-NH-(CH₂)y-N(CH₃)₂)-(CH₂)z-O-(CH₂)p]n-, with x, y, z, p=1-12, and n according to the molecular weight requirements. Examples are Polyquaternium 2 (Mirapol A-15), Polyquaternium-17 (Mirapol AD-1), and Polyquaternium-18 (Mirapol AZ-1).

Other polymers include cationic polysiloxanes and cationic polysiloxanes with carbon-based grafts with a net theoretical positive charge or equal to zero (mixture of cationic and anionic functional groups). This includes cationic end-group functionalized silicones (i.e. Polyquaternium-80). Silicones with general structure: [-Si (R1) (R2) -O-] x- [Si (R3) (R2) -O-] y-where R1 is any alkane from C1-C25 or H with number of double bonds from 0-5,aromatic moieties, polysiloxane grafts, or mixtures thereof. R1 can also be a liquid crystalline moiety that can render the polymer thermotropic liquid crystalline properties, or the alkanes selected can result in side-chain melting. R2 can be H or CH3 and R3 can be -R1-R4, where R4 can be -NH₂, -NHR1, -NR1R2, -NR1R2R6 (where R6 = R1, R2, or -CH₂-COOH or its salt), -NH-C(O)-, -COOH, -COO- alkali salt, any C1-25 alcohol, -C(O)-NH₂ (amide), -C(O)-N(R2)(R2')(R2"), sulfo betaine, betaine, polyethylene oxide, poly(ethyleneoxide/propylene oxide/butylene oxide) grafts with any end group, H, -OH, styrene sulfonate, pyridine, quaternized pyridine, alkyl-substituted pyrrolidone or pyridine, pyridine-N-oxide, imidazolinium halide, imidazolium halide, imidazol, piperidine, pyrrolidone, caprolactam, -COOH, -COO- alkali salt, sulfonate, ethoxy sulphate phenyl-R5 or naphthalene-R6 where R5 and R6 are R1, R2, R3, sulfonic acid or its alkali salt or organic counter ion. R3 can also be -(CH₂)x-O-CH₂-CH(OH)-CH₂₋N(CH₃)₂-CH₂-COOH and its salts. Any mixture of these R3 groups can be selected. X and y can be varied as long as the theoretical net charge of the polymer is zero (amphoteric) or positive. In addition, polysiloxanes containing up to 5 different types of monomeric units may be used. Examples of suitable polysiloxanes are found in U.S. Patent Nos. 4,395,541 4,597,962 and U.S.6,200,554. Another group of polymers that can be used to improve capsule/particle deposition are phospholipids that are modified with cationic polysiloxanes. Examples of these polymers are found in U.S. Patent No. 5,849,313, WO Patent Application 9518096A1 and European Patent EP0737183B1.

Furthermore, copolymers of silicones and polysaccharides and proteins can be used (commercially available as CRODASONE brand products).

Another class of polymers include polyethylene oxide-co-propyleneoxide-co-butylene oxide polymers of any ethylene oxide/propylene oxide / butylene oxide ratio with cationic groups resulting in a net theoretical positive charge or equal to zero (amphoteric). The general structure is: 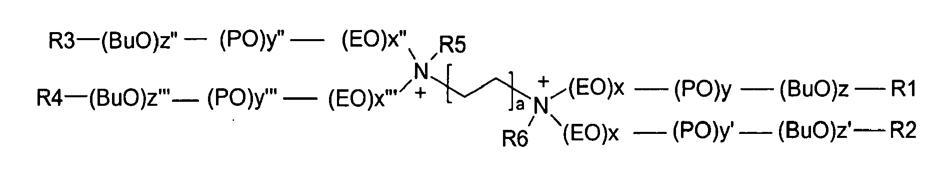 where R1,2,3,4 is -NH2, -N(R)3- X+, R with R being H or any alkyl group. R5, 6 is -CH3 or H. The value for 'a' can range from 1-100. Counter ions can be any halide ion or organic counter ion. X, Y, may be any integer, any distribution with an average and a standard deviation and all 12 can be different. Examples of such polymers are the commercially available TETRONIC brand polymers.

Suitable polyheterocyclic (the different molecules appearing in the backbone) polymers include the piperazine-alkylene main chain copolymers disclosed in Ind. Eng. Chem. Fundam., (1986), 25, pp.120-125, by Isamu Kashiki and Akira Suzuki.

Also suitable for use in the present invention are copolymers containing monomers with cationic charge in the primary polymer chain. Up to 5 different types of monomers may be used. Any co-monomer from the types listed in this specification may also be used. Examples of such polymers are poly diallyl dimethyl ammonium halides (PolyDADMAC) copolymers of DADMAC with vinyl pyrrolidone, acrylamides, imidazoles, imidazolinium halides, etc. These polymers are disclosed in Henkel EP0327927A2 and PCT Patent Application 01/G2376A1. Also suitable are Polyquaternium-6 (Merquat 100), Polyquaternium-7 (Merquats S, 550, and 2200), Polyquaternium-22 (Merquats 280 and 295) and Polyquaternium-39 (Merquat Plus 3330), available from Ondeo Nalco.

Polymers containing non-nitrogen cationic monomers of the general type -CH2-C(R1) (R2-R3-R4)- can be used with:
R1 being a -H or C1-C20 hydrocarbon. R2 is a disubstituted benzene ring or an ester, ether, or amide linkage. R3 is a C1-C20 hydrocarbon, preferably C1-C10, more preferably C1-C4. R4 can be a trialkyl phosphonium, dialkyl sulfonium, or a benzopyrilium group, each with a halide counter ion. Alkyl groups for R4 are C1-C20 hydrocarbon, most preferably methyl and t-butyl. These monomers can be copolymerized with up to 5 different types of monomers. Any co-monomer from the types listed in this specification may also be used.

Substantivity of these polymers may be further improved through formulation with cationic, amphoteric and nonionic surfactants and emulsifiers, or by coacervate formation between surfactants and polymers or between different polymers. Combinations of polymeric systems (including those mentioned previously) may be used for this purpose as well as those disclosed in EP1995/000400185.

Furthermore, polymerization of the monomers listed above into a block, graft or star (with various arms) polymers can often increase the substantivity toward various surfaces. The monomers in the various blocks, graft and arms can be selected from the various polymer classes listed in this specification and the sources below:
Encyclopedia of Polymers and Thickeners for Cosmetics, Robert Lochhead and William From, in Cosmetics & Toiletries, Vol. 108, May 1993, pp. 95-138;
Modified Starches: Properties & Uses, O. B. Wurzburg, CRC Press, 1986. Specifically, Chapters 3, 8, and 10;
U.S. Patent Nos. 6,190,678 and 6,200,554; and
PCT Patent Application WO 01/62376A1 assigned to Henkel. Polymers, or mixtures of the following polymers:
   (a) Polymers comprising reaction products between polyamines and (chloromethyl) oxirane or (bromomethyl) oxirane. Polyamines being 2(R1)N-[-R2-N(R1)-]n-R2-N(R1)2, 2HN-R1-NH2, 2HN-R2-N(R1)2 and 1H-Imidazole. Also, the polyamine can be melamine. R1 in the polyamine being H or methyl. R2 being alkylene groups of C1-C20 or phenylene groups. Examples of such polymers are known under the CAS numbers 67953-56-4 and 68797-57-9. The ratio of (chloromethyl) oxirane to polyamine in the cationic polymer ranges from 0.05-0.95.
   (b) Polymers comprising reaction products of alkanedioic acids, polyamines and (chloromethyl) oxirane or (bromomethyl) oxirane. Alkane groups in alkanedioic acids C0-C20. Polyamine structures are as mentioned in (a). Additional reagents for the polymer are dimethyl amine, aziridine and polyalkylene oxide (of any molecular weight but, at least, di-hydroxy terminated; alkylene group being C1-20, preferably C2-4). The polyalkylene oxide polymers that can also be used are the Tetronics series. Examples of polymers mentioned here are known under the CAS numbers 68583-79-9 (additional reagent being dimethyl amine), 96387-48-3 (additional reagent being urea), and 167678-45-7 (additional reagents being polyethylene oxide and aziridine). These reagents can be used in any ratio.
   (c) Polyamido Amine and Polyaminoamide-epichlorohydrin resins, as described by David Devore and Stephen Fisher in Tappi Journal, vol.76, No.8, pp. 121-128 (1993). Also referenced herein is "Polyamide-polyamine-epichlorohydrin resins" by W. W. Moyer and R. A. Stagg in Wet-Strength in Paper and Paperboard, Tappi Monograph Series No. 29, Tappi Press (1965), Ch.3, 33-37.

The preferred cationically charged materials comprise reaction products of polyamines and (chloromethyl) oxirane. In particular, reaction products of 1H-imidazole and (chloromethyl) oxirane, known under CAS number 68797-57-9. Also preferred are polymers comprising reaction products of 1,6-hexanediamine,N-(6-aminohexyl) and (chloromethyl) oxirane, known under CAS number 67953-56-4. The preferred weight ratio of the imidazole polymer and the hexanediamine, amino hexyl polymer is from about 5:95 to about 95:5 weight percent and preferably from about 25:75 to about 75:25.

The level of outer cationic polymer is from about 1% to about 3000%, preferably from about 5% to about 1000% and most preferably from about 10% to about 500% of the fragrance containing compositions, based on a ratio with the fragrance on a dry basis.

The weight ratio of the encapsulating polymer to fragrance is from about 1:25 to about 1:1. Preferred products have had the weight ratio of the encapsulating polymer to fragrance varying from about 1:10 to about 4:96.

For example, if a capsule blend has 20 weight % fragrance and 20 weight % polymer, the polymer ratio would be (20/20) multiplied by 100 (%) = 100%.

According to the present invention, the encapsulated fragrance is well suited for wash-off products. Wash-off products are understood to be those products that are applied for a given period of time and then are removed. These products are common in areas such as laundry products, and include detergents, fabric conditioners, and the like; as well as personal care products which include shampoos, conditioner, hair colors and dyes, hair rinses, body washes, soaps and the like.

As described herein, the present invention is well suited for use in a variety of well-known consumer products such as laundry detergent and fabric softeners, liquid dish detergents, automatic dish detergents, as well as hair shampoos and conditioners. These products employ surfactant and emulsifying systems that are well known. For example, fabric softener systems are described in U.S. Patents 6,335,315, 5,674,832, 5,759,990, 5,877,145, 5,574,179, 5,562,849, 5,545,350, 5,545,340, 5,411,671, 5,403,499, 5,288,417, 4,767,547 and 4,424,134. Liquid dish detergents are described in 6,069,122 and 5,990,065; automatic dish detergent products are described in 6,020,294, 6,017,871, 5,968,881, 5,962,386, 5,939,373, 5,914,307, 5,902,781, 5,705,464, 5,703,034, 5,703,030, 5,679,630, 5,597,936, 5,581,005, 5,559,261, 4,515,705, 5,169,552, and 4,714,562. Liquid laundry detergents which can use the present invention include those systems described in 5,929,022, 5,916,862, 5,731,278, 5,565,145, 5,470,507, 5,466,802, 5,460,752, 5,458,810, 5,458,809, 5,288,431,5,194,639, 4,968,451, 4,597,898, 4,561,998, 4,550,862, 4,537,707, 4,537,706, 4,515,705, 4,446,042, and 4,318,818. Shampoo and conditioners that can employ the present invention include 6,162,423, 5,968,286, 5,935561, 5,932,203, 5,837,661, 5,776,443, 5,756,436, 5,661,118, 5,618,523, 5,275,755, 5,085,857, 4,673,568, 4,387,090, 4,705,681.

We have discovered that the present invention is advantageously applied to products, including fabric rinse conditioners, having a pH of less than 7, preferably less than about 5 and most preferably less than about 4.

A better product, including wash-off products such as fabric rinse conditioner is also obtained when the salt level is limited. The improvement in the fabric rinse conditioner is noted by a longer lasting and/or improved delivery of fragrance. One method of improving the delivery of the encapsulated fragrance is to limit the amount of salt in the product base. Preferably the level of salt in the rinse conditioner product is less than or equal to about 1 weight percent by weigh in the product, preferably less than about 0.5 weight percent and most preferably less than about 0.1 weight percent.

More specifically we have discovered that limiting the level of calcium chloride will improve the delivery of the fragrance using the encapsulated fragrance of the present invention. Improved fragrance delivery is provided by limiting the amount of calcium chloride to less than about 2 weight percent, typically less than 1 weight percent and more preferably less than 0.5 weight percent. As it is known in the art, calcium chloride is added to control viscosity of the formulations, so there is trade-off between the viscosity and fragrance delivery. We have discovered that limiting the level of calcium chloride level as set forth above is particularly advantageous in fabric rinse conditioner products.

Another means for improving the performance of delivery of the encapsulated fragrance of the present invention is to limit the level of some softening agents. We have discovered that limiting the softening actives, such as triethanolamine quaternary, diethanolamine quaternary, ACCOSOFT cationic surfactants (Stepan Chemical), or ditallow dimethyl ammonium chloride (DTDMAC), to an amount of from about 5 to about 40 weight percent of the product, preferably from about 5 to about 30 and more preferably from about 5 to 15 weight percent of a fabric rinse conditioner product will improve the performance of the fragrance. The above softening agents are well known in the art and are disclosed in U.S. Patents 6,521,589 and 6,180,594.

Yet another means for improving fragrance delivery of the present invention is to limit the level of the non-ionic surfactants employed in the product, including a fabric softening product. Many non-ionic surfactants are known in the art and include alkyl ethoxylate, commercially available as NEODOL (Shell Oil Company), nonyl phenol ethoxylate, TWEEN surfactants (ICI Americas Inc.), and the like. We have discovered that the encapsulated fragrance of the present invention are advantageously used when the non-ionic surfactant level is below about 5 weight percent of the product, preferably less than about 1 weight percent and most preferably less than 0.5 weight percent.

Yet another means for enhancing the fabric softener product is to limit the level of co- solvent included in the fabric softener in addition to water. Reducing the level of co solvents such as ethanol and isopropanol to less than about 5 weight percent of the product, preferably less than about 2 and most preferably less than about 1 weight percent of the fabric softener product has been found to improve fragrance delivery.

Improved fragrance performance includes longer lasting fragrance, improved substantivity of the fragrance on cloth or the ability to provide improved fragrance notes, such as specific fragrance notes through the use of the present invention.

While the above description is primarily to fabric rinse conditioner products, additional studies for shampoos, detergent and other cleaning products have also led to preferred embodiments for these products as well.

As was found for fabric rinse conditioners, additional studies have determined that lower pH is desirable for the delivery of fragrance when used in the product base. The preferred bases are neutral or mildly acidic, preferably having a pH of 7, more preferably less than about 5 and most preferably less than about 4 for shampoos, detergent and other cleaning products.

We have found that powder detergent and other cleaning products provide enhanced fragrance delivery when the material coating the encapsulating polymer is also neutral or slightly acidic. Preferred materials are NaHSO4, acetic acid, citric acid and other similar acidic materials and their mixtures. These materials have a pH of less than about 7, preferably less than about 5 and most preferably less than about 4.

As was described with fabric rinse conditioners, lower surfactant levels were advantageously employed in shampoos, detergents and other cleaning products bases with the present invention. The level of surfactant is preferably less than about 30, more preferably less than about 20 and most preferably less than about 10 weight percent of the product base. A similar finding was found with preferred levels of salt in shampoos, detergents and other cleaning products as was found in fabric rinse conditioners. The salt level is preferably less than about 5 weight percent, more preferably less than about 2 and most preferably less than 0.5 weight percent of the product.

Lower solvent levels found in the base improves the fragrance delivery in shampoos, detergents and other cleaning products as well. Solvents, include but are not limited to, ethanol, isopropanol, dipropylene glycol in addition to the water base and the hydrotope level is preferably less than 5 weight percent, preferably less than about 2 and most preferably less than 1 weight percent of the total product base.

A preferred surfactant base for shampoos, detergents and other cleaning products was found to be ethoxylated surfactants such as alkyl ethoxylated sulfates, (C₁₂-C₁₄) (ethylene oxide) nSO₄M; or ethoxylated carboxylate surfactants (C₁₂₋C₁₄)(Ethylene oxide)nCOOM where n is from 1 to about 50 and M is Na⁺, K⁺ or NH4⁺ cation. Other preferred anionic surfactants are alkoyl isethionates, such as sodium cocoly isethionate, taurides, alpha olefin sulphonates (i.e., Bioterge, Stepan Corporation), sulfosuccinates, such as Standapol SH-100 (Cognis) and disodium laureth sulfosuccinate (Stepan Mild SL3-BA, Stepan Corporation). A more preferred class of surfactants for use in the present invention was zwitterionic surfactants such as the alkyl amine oxides, amidealkyl hydroxysultaines like amidopropyl hydroxyl sultaine (Amphosol CS-50, Stepan Corporation), amphoacetates, such as sodium cocamphoacetate (Amphosol IC, Stepan Corporation), betaines and sulfobetaines. Zwitterionic surfactants are disclosed in greater detail in U.S. Patent 6,569,826. Other commercially available surfactants are AMPHOSOL series of betaines (Stepan Chemical); TEGOTIAN by Goldschmidt; and HOSTAPAN and ARKOPAN by Clariant

The most preferred surfactant system to be employed with the encapsulated fragrance system of the present invention was found to be non-ionic surfactants. Nonionic surfactants that may be used include the primary and secondary alcohol ethoxylates, especially the C₈-C₂₀ aliphatic alcohols ethoxylated with an average of from 1 to 50 moles of ethylene oxide per mole of alcohol, and more especially the C₁₀-C₁₅ primary and secondary aliphatic alcohols ethoxylated with an average of from 1 to 10 moles of ethylene oxide per mole of alcohol. Other ethoxylated nonionic surfactants that are suitable are polyethylene glycol (MW=200-6000) esters of fatty acids, ethylene oxide-propylene oxide-butylene oxide block copolymers such as the Pluronic and Tetronic polymers made by BASF, and ethoxylated alkanolamides such as PEG-6 cocamide (Ninol C-5, Stepan Corporation). Non-ethoxylated nonionic surfactants include alkylpolyglycosides, glycerol monoethers, polyhydroxyamides (glucamide), polyglycerol fatty acid esters, alkyl pyrrolidone-based surfactants (Surfadone LP-100 and LP300, ISP Corporation), dialkyl phthalic acid amides (distearyl phthalic acid amide or Stepan SAB-2 by Stepan Corporation), alkyl alkanolamides, such as Laureth Diethanolamide (Ninol 30-LL, Stepan Corporation). These nonionic surfactants are disclosed in U.S. Patent 6,517,588.

In addition, Gemini surfactants can be used, such as the Gemini polyhydroxy fatty acid amides disclosed in U.S. Patent 5,534,197. Furthermore, structured liquids can be used that contain lamellar vesicles or lamellar droplets, as disclosed in WO 9712022 A1 and WO 9712027 A1, 5,160,655, and 5,776,883.

The rinse-off products that are advantageously used with the polymer encapsulated fragrance of the present invention include laundry detergents, fabric softeners, bleaches, brighteners, personal care products such as shampoos, conditioners, hair colors and dyes, rinses, creams, body washes and the like. These may be liquids, solids, pastes, or gels, of any physical form. Also included in the use of the encapsulated fragrance are applications where a second active ingredient is included to provide additional benefits for an application. The additional beneficial ingredients include fabric softening ingredients, skin moisturizers, sunscreen, insect repellent and other ingredients as may be helpful in a given application. Also included are the beneficial agents alone, that is without the fragrance.

While the preferred coating materials may be simply dissolved in water and mixed with a suspension of capsules prior to addition to the final product, other modes of coating use and application are also possible. These modes include drying the coating solution in combination with the capsule suspension for use in dry products such as detergents, or using higher concentrations of coating such that a gel structure is formed, or combining the coating material with other polymers or adjuvants which serve to improve physical characteristics or base compatibility. Drying or reducing the water content of the capsule suspension prior to coating addition is also possible, and may be preferable when using some coating materials. Further, when using some coating materials it is possible to add the coating to the application base separately from the encapsulated fragrance.

Solvents or co-solvents other than water may also be employed with the coating materials. Solvents that can be employed here are (i) polyols, such as ethylene glycol, propylene glycol, glycerol, and the like, (ii) highly polar organic solvents such as pyrrolidine, acetamide, ethylene diamine, piperazine, and the like, (iii) humectants/plasticizers for polar polymers such as monosaccharides (glucose, sucrose, etc.), amino acids, ureas and hydroxyethyl modified ureas, and the like, (iv) plasticizers for less polar polymers, such as diisodecyl adipate (DIDA), phthalate esters, and the like.

Rheology modifiers should be selected carefully to insure compatibility with the deposition agents. Preferred are nonionic, cationic and amphoteric thickeners, such as modified polysaccharides (starch, guar, celluloses), polyethylene imine (Lupasol WF, BASF Corporation), acrylates (Structure Plus, National Starch and Chemical Company) and cationic silicones.

The coating polymer(s) may also be added to a suspension of capsules that contain reactive components such that the coating becomes chemically (covalently) grafted to the capsule wall, or the coating polymer(s) may be added during the crosslinking stage of the capsule wall such that covalent partial grafting of the coating takes place.

Further, if stability of the capsule and coating system is compromised by inclusion in the product base, product forms which separate the bulk of the base from the fragrance composition may be employed. The cationic coated polymer particles of the present invention may be provided in solid and liquid forms depending on the other materials to be used. In order to provide the cationic coated polymer in a dry form, it is preferable that the materials be dried using drying techniques well known in the art. In a preferred embodiment the materials are spray dried at the appropriate conditions. The spray dried particles may also be sized to provide for consistent particle size and particle size distribution. One application in which it would be advantageous to include dry particles of the present invention would be incorporated in a powdered laundry detergent. Alternatively wet capsule-coating slurries may be absorbed onto suitable dry powders to yield a flowable solid suitable for dry product use.

The present invention also includes the incorporation of a silicone or a siloxane material into a product that contains encapsulated fragrances of the present invention. As used herein silicone is meant to include both silicone and siloxane materials. Also included in the definition of silicone materials are the cationic and quaternized of the silicones. These materials are well known in the art and include both linear and branched polymers.

In addition to silicones, the present invention also includes the use of mineral oils, triglyceride oils, polyglycerol fatty acid esters, and sucrose polyester materials in a similar matter as the silicone materials. For brevity, these materials are understood to be included in the term silicone as used in this specification unless noted to the contrary. Those with skill in the art will also appreciate that it is possible to incorporate a silicone in combination with mineral oils and the like in carrying out the present invention.

The silicone material is preferably admixed to the encapsulated active material-containing product after the active materials are encapsulated. Optionally, the silicone material may be mixed directly with the product base either before or after the encapsulated material has been added.

Suitable silicone materials include amodiemthicone, polymethylalkyl siloxanes, polydimethylalkyl siloxanes, dimethicone, dimethicone copolyol, dimethiconol, disiloxane, cyclohexasiloxane, cyclomethicone, cyclopentasiloxane, phenyl dimethicone, phenyl trimethicone, silicone quaternarary materials including silicone quaternium-8, and silicone quaternium-12, trimethylsiloxyamidodimethicone, trimethylsiloxysilicate and the like. These materials are commercially well known materials and are available from suppliers such as Dow Corning, Shin-Etsu, Wacker Silicones Corporation and the like. The preferred silicon is Dow Corning 245 Fluid (Dow Corning, Midland Michigan), which is described as containing greater than about 60 weight percent decamethylcyclopentasiloxane and less than or equal to about 4 weight percent dimethylcyclosiloxanes.

Amino functional silicone oils such as those described in U.S. Patents 6,355,234 and 6,436,383 may also be used in the present invention.

Preferably the silicone materials of the present invention have a molecular weight (Mw) of from about 100 to about 200,000, preferably from about 200 to about 100,00 and most preferably from about 300 to about 50,000.

The viscosity of the silicone materials is typically from 0.5 to about 25, preferably from about 1 to about 15 and most preferably from about 2 to about 10 millimeters²sec-1 using the Corporate Test Method as described in the Dow Corning product brochures.

The level of silicone used in the present invention varies by product, but is typically less than 10 percent by weight, typically from about 0.5 to about 8 weight percent of the total weight of product. Preferably the silicon level is from about 2 to about 6 and most preferably from about 3 to about 5 weight percent of the total weight of the product.

The silicone fluid can be added to a wide array of products in order to enhance the delivery of fragrance. Suitable products include fabric conditioners and detergents, personal care products such as shampoos, liquid soap, body washes and the like; as well as in applications such as fine fragrances and colognes.

In another embodiment of the present invention, we have discovered that the cationic coating is not required and that the inclusion of silicon in the encapsulated mixture can provide satisfactory performance in the delivery of the fragrance. In this embodiment of the invention, the fragrance is encapsulated by the polymeric materials described above, and the level of silicon described above is provided to the encapsulated fragrance.

More specifically the present invention is directed to a composition comprising an active material, said active material encapsulated by a polymer to provide a polymer encapsulated material, said polymer encapsulated material further provided with a silicone material. This embodiment differs from other embodiments of the present invention in that the cationic polymer is not provided. The silicone oil is provided without a cationic polymer present. A description of the suitable silicone oils is provided above as well as the level of the silicon oil that is used.

The mixture mentioned above can be provided into a wide range of products, including rinse-off products including but not limited to fabric rinse conditioners, detergents, shampoos, conditioners, hair color and dyes, body washes, and other cleaning products such as dryer tumbler sheets.

It should be noted that the cationic character of the polymer coating used is not sufficient to determine whether it is functional with regard to improving capsule or particle deposition. Without wishing to be bound by theory, it is hypothesized that while cationic charge provides an affinity to the normally anionic substrates of interest (i.e. hair, skin, and cloth), other physical characteristics of the polymer are also important to functionality. Additionally, interactions between the capsule or particle surface, base ingredients, and the coating polymer are thought to be important to improving deposition to a given substrate.

Use of the coating systems described below allows for more efficient deposition of capsules, particles, and dispersed droplets that are coated by the cationically charged polymer. Without wishing to be bound by any theory it is believed that the advantages of the present invention is created by the combination of the cationically charged coating which is helpful in adhering to the substrate to which the product is applied with a capsule or particle containing active material and/or odor controlling material. Once the encapsulated particle is adhered to the substrate we have found that the encapsulated material can be delivered by the fracturing or compromising of the polymer coating by actions such as brushing hair, movement of the fabric, brushing of the skin etc.

One measurement of the enhancement of the present invention in delivering the fragrance and other ingredients of the present invention is done by headspace analysis. Headspace analysis can provide a measure of the fragrance material contained on the desired substrate provided by the present invention. The present invention will provide a much higher level of fragrance on the substrate compared to the amount of fragrance deposited on the substrate by conventional means. As demonstrated by the following examples, the present invention can deliver more than about twice the level of fragrance to a substrate than common approaches, preferably more than about three times the level of fragrance and preferably more than about five times the level of fragrance than traditional approaches.

For example, this may be determined by measuring the level of fragrance imparted to a test hair swatch containing fragrance in a shampoo by conventional means as compared to the level of fragrance imparted by the present invention. The same fragrance should be used and similar test hair pieces should be washed in a similar manner. After brushing to release the fragrance from the hair, the level of fragrance on the test hair swatches of the control and the fragrance of the present invention could be measured by headspace analysis. Due to the superior adhesion of fragrance to hair by the present invention, the headspace analysis of the respective samples will demonstrate an improved level of fragrance as compared to fragrance applied by conventional means.

To better control and measure the fragrance release upon brushing or rubbing from a substrate (i.e., hair or cotton cloth), a fixed-weight of the washed and dried substrate will be placed in a custom-made glass vessel containing SILCOSTEEL (Resteck Corp., Bellefont, PA) treated steel ball bearings. Headspace will be collected from the vessel using a Tenax trap (Supelco, Inc., Bellafonte, PA) upon equilibration. A second headspace will be collected after the substrate-containing vessel is shaken along with the steel beads on a flat bed shaker for 20 minutes. Fragrance present in the headspace from unshaken and shaken substrates and subsequently absorbed in the Tenax traps is desorbed through a Gerstel thermal desorption system (Gersteel, Inc., Baltimore, MD). Desorbed fragrance volatiles are injected into a gas chromatograph (Hewlett-Packard, Model Agilent 6890) equipped with a flame ionization detector. Area counts of individual fragrance components, identified based on the retention time, are then collected and analyzed. See commonly assigned US Application 10/753,847.

In another embodiment of the present invention, after the wall is formed and the reaction between the polymer and crosslinker is completely reacted the encapsulated active material and/or odor controlling material can be further crosslinked, referred to as secondary crosslinking, by adding an appropriate crosslinker and modifying the external aqueous environment to facilitate the secondary crosslinking reaction.

With respect to the primary crosslinker, wall properties are influenced by two factors: the degree of crosslinking and the hydrophobic or hydrophilic nature of the crosslinker. The quantity and reactivity of the crosslinker determine the degree of crosslinking. The degree of crosslinking influences the capsule wall permeability by forming physical barriers towards diffusion. Walls made from crosslinkers possessing low-reactive groups will have smaller degrees of crosslinking than walls made from high-reactive crosslinkers. If a high degree of crosslinking is desired from a low-reactive crosslinker, more is added. If a low degree of crosslinking is desired from a high-reactive crosslinker then less is added. The nature and quantity of the crosslinker can also influence the hydrophobicity/hydrophilicity of the wall. Some crosslinkers are more hydrophobic than others and these can be used to impart hydrophobic qualities to the wall, with the degree of hydrophobicity directly proportional to the quantity of crosslinker used.

The degree of crosslinking and degree of hydrophobicity can result from a single crosslinker or a combination of crosslinkers. A crosslinker that is highly reactive and hydrophobic can be used to create capsule walls with a high degree of crosslinking and a hydrophobic nature. Single crosslinkers that possess both these qualities are limited and thus crosslinker blends can be employed to exploit these combinations. Crosslinkers possessing high reactivities but low hydrophobicities can be used in combination with a low reactive, high hydrophobicity crosslinker to yield walls with high degrees of crosslinking and high hydrophobicity.

Secondary crosslinking also allows the introduction and use of certain crosslinkers that are not active in the initial capsule forming reaction. It allows additional characteristics to be applied to the capsule wall. Secondary crosslinking can form an exterior seal or coating on the active material and/or odor controlling material microcapsule which can prevent active material loss via leaching. This exterior coating can also act as a deposition aid by modifying the capsule surface to increase the affinity towards various substrates. In this way wall properties can be ultimately changed.

Microcapsule slurries consist of microcapsules containing active materials dispersed in aqueous medium. The microcapsules themselves are not resistant to pH or temperature extremes. These environmental constraints limit the scope of available reactions that can be performed on the microcapsule wall. Two classes of crosslinker are capable of effecting transformations in this environment: aminoplasts and aldehydes. Aminoplast chemistry is simply an extension of the chemistry used to form the microcapsule wall, producing ester, ether, and imino bonds. The aldehyde chemistry follows a different mechanism and results in Schiff base/imine formation with amines.

The functional groups present in the wall govern which type of crosslinker can be used. Amides, carboxyls, hydroxyls, thiols, and amines respond well to aminoplast types of crosslinkers such as melamine-formaldehyde, urea-formaldehyde and glycourils. Amine groups react well with aldehydes, such as glutaraldehyde, formaldehyde, phthalidicarboxaldehyde, as well as with tannins/tannic acid and dihydroxyacetone. Aminoplasts and aldehydes may be used in combination when the wall consists of solely amine groups or a mixture of amines and aminoplast reactive groups such as carboxyls.

In addition to the di- and tri- functional crosslinking agents above, mono-functional species can be utilized as well. In this case the purpose is not crosslinking, but endcapping. Endcapping certain moieties on the capsule wall can change the exterior character of the capsule by introducing hydrophobic groups or by masking the native moieties and preventing undesirable interactions. For capsule walls containing aminoplast species any mono-functional amine, alcohol, carboxylic acid, or thiol can be used. Capsule walls that possess amides, carboxyls, hydroxyls, thiols, and amines can be endcapped with mono-functional amino-formaldehyde adducts. Capsule walls containing amines can be endcapped with monofunctional aldehydes such as acetaldehyde or benzaldehyde.

The secondary crosslinking can occur during the capsule making reaction or after the reaction is complete. If the secondary crosslinking is to occur during the reaction it can happen as usual during curing or as an additional step at the end. Alternatively the secondary crosslinking can occur after the reaction is complete. This can happen immediately afterwards or up to days or weeks later. If there are any additives such as stabilizers or formaldehyde scavengers that are used at the end of the process it is important to postpone their use until the secondary crosslinking is complete.

Aminoplast crosslinkers can be employed at levels ranging from fractions of the original use weight to several times the use rate. The level chosen depends on the desired effect. At lower levels below a certain point the benefit is minimized or non existent. At higher levels the additional crosslinking increases the wall strength of the capsule so that breakage, and hence fragrance release, is impossible. Typical secondary aminoplast crosslinker levels are between 50% and 300% times the primary level. Aldehyde crosslinkers are employed according to the amino group content of the wall. They are added at levels ranging from 50% to 2% the calculated amino group level. At levels above 50% there is no additional benefit since there will not be enough amino groups to crosslink. At levels below 2% the effect of crosslinking is not observed.

Aminoplast crosslinkers require weakly basic to moderately acidic pH's for reactivity (pH 3 to 8), depending on the aminoplast and functional groups involved in the reaction. Melamine-formaldehyde crosslinkers are active from pH 3 to pH 8. For reaction with melamine-formaldehyde crosslinkers amine functional groups require pH 8 whereas carboxyl groups are active at pH 5. Urea-formaldehyde and glycouril crosslinkers are active at pH 3. Aldehyde crosslinkers are reactive from acidic to basic pHs depending on the type. Glutaraldehyde is active towards amines at all pHs whereas formaldehyde is only active at basic pH's. Tannic acid is active at neutral pH and dihydroxyacetone at basic pH's. In all instances the microcapsule must be able to withstand the pH changes.

A further embodiment of the invention is directed to a process for improving the performance and stability of encapsulated fragrances by catalyzing the curing crosslinking reaction during capsule formation thereby providing improved capsule formation.

According to the present invention the capsule process can be catalyzed by acids and/or metal salts and mixtures thereof, to produce better performance and stability in a base, such as, but not limited to, a fabric conditioner, dry and liquid detergent, tumbler dryer sheets, shampoo, body lotion, body wash, hard surface cleaners, soap bars, hair conditioners, hair fixatives, hair color and dyes and after-shave lotions. In additional these capsules may be applied (physical or chemically) on textile fabrics during manufacture.

Performance, as defined herein, relates to fragrance intensity of the encapsulated samples versus the neat as determined by sensory response or analytical headspace as the capsule-containing base is aged at 37°C. Stability, as previously defined, is the constant capsule slurry viscosity over time.

The role of the acid catalyst is two-fold: first it causes the prepolymer to build viscosity, which is necessary for capsule formation, and second, it catalyzes the curing crosslinking reaction. The standard acid catalyst used in the current process known in the art is acetic acid at a level of 6 to 7 % trs (total resin solid), which results in a pH of 5.0.

As discussed above, the rate of the viscosity buildup of the prepolymer is a function of pH, with the rate increasing as a function of decreasing pH. The pH may be adjusted using the acid catalyst. The catalysts may be both weak and strong organic and mineral acids.

Examples of acid catalyst include, but are not limited to, hydrochloric (HCl), sulfuric, phosphoric, *para*-toluenesulfonic (pTSA), acetic, glycolic, lactic, benzoic, citric, maleic, and commercially available catalysts from the coatings and textile industries (Nacure XP-333 (available from King Industries), K-Cure 129W (available from King Industries), Cycat 296-9 (available from Cytec), Polystep A-13 (available from Stepan).

In addition to affecting the prepolymer viscosity buildup, these acids also affect the capsule performance by participating in the crosslinking reaction, acting as crosslinkers themselves. The aforementioned acids can be grouped according to their functionalities: monoprotic, diprotic, and triprotic. Examples of suitable diprotic acids are oxalic and maleic. A trifunctional acid includes citric acid.

Capsule performance is sensitive to the degree of crosslinking that the multifunctional acids impart. Therefore it may be necessary to adjust these acid levels up or down to optimize performance.

In addition to acids, metal salts can also be used to enhance capsule stability and performance, alone or in combination with the acid catalysts. By themselves the metal salts act as Lewis acids which enhance the crosslinking. In combination with alpha-hydroxy acids, such as, citric, glycolic, lactic, there is an enhanced catalytic effect due to the metal salt's coordination with the hydroxyl group on the acid. These salts can be employed in levels that range from about 1 to about 15% trs.

The metal salts can be selected from the group consisting of but not limited to ammonium chloride (NH₄Cl), aluminum nitrate (Al(NO₃)₃), aluminum sulfate (Al(SO₄)₃), magnesium bromide (MgBr₂), magnesium chloride (MgCl₂), magnesium nitrate (Mg(NO₃)₂), zinc bromide (ZnBr₂), zinc iodide (ZnI₂), and zirconyl nitrate (ZrO(NO₃)₂). Preferred metal salts include NH₄Cl , MgBr₂, MgCl₂, Mg(NO₃)₂, and ZnI₂. More preferred metal salts include MgBr₂, MgCl₂, Mg(NO₃)₂, and ZnI₂ result in performance gains.

In a further embodiment, alpha-hydroxyacids such as lactic, glycolic, and citric lactic performance have a synergistic effect with metal salts such as, but not limited to MgBr₂, MgCl₂, Mg(NO₃)₂, ZnI₂ and mixtures thereof.

All U.S. Patents and patent applications cited herein are incorporated by reference as if set forth herein in their entirety.

These and additional modifications and improvements of the present invention may also be apparent to those with ordinary skill in the art. The particular combinations of elements described and illustrated herein are intended only to represent only a certain embodiment of the present invention and are not intended to serve as limitations of alternative articles within the spirit and scope of the invention. All materials are reported in weight percent unless noted otherwise. As used herein all percentages are understood to be weight percent.

### Example I

### Naturally-derived amine containing polymer

6.8g of gelatin (Type A, 300 bloom) and 320.2g water were combined and heated to between 50 and 60°C until the gelatin dissolved. 18g of Cymel 385 were then added and the mixture stirred until clear. The pH was adjusted to 4 with 10M HCl. 134g of core material containing 67g of fragrance oil and 67g of modifier (Neobee M-5 oil) were emulsified until the particle size was between 10 and 20 µm. The emulsion was then heated to 80°C and held at 80°C for 2 hours. After cooling fragrance microcapsules were obtained. The mean capsule size was 12.7 µm and the encapsulation efficiency was 96.50%. The capsules were incorporated in fabric conditioner. Cloths washed with this fabric conditioner exhibited enhanced fragrance levels and burst effects compared to cloths washed with neat fragrance.

### Example II

### Synthetic amine containing polymer

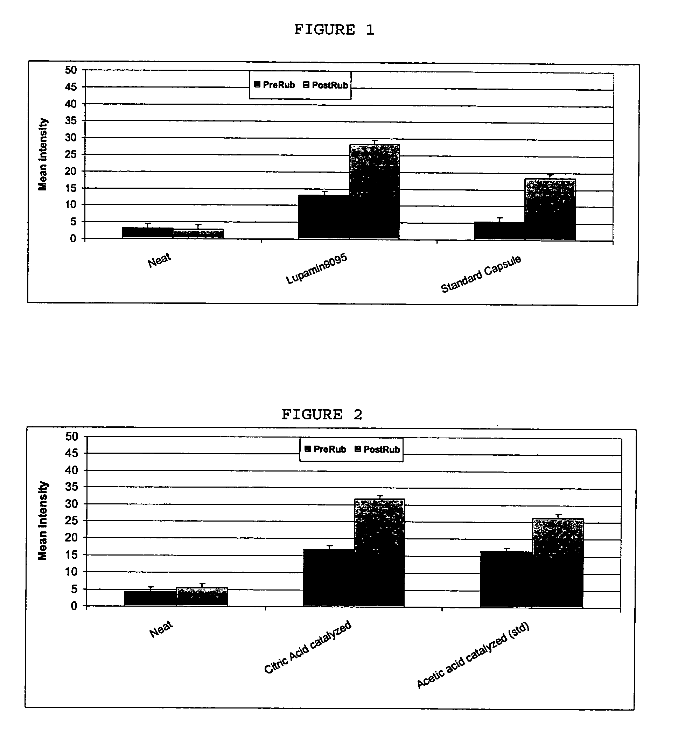

34g of Lupamin 9095, 18g Cymel 385 (available from Cytec), and 293g water were combined and stirred until dissolved. The pH was left in a natural state at about 8. The mixture was held at 50°C for approximately 135 minutes, at which time 168g of core material containing 84g of fragrance oil and 84g of modifier (Neobee M-5 oil) were added. The mixture was emulsified until the particle size was between 10 and 20 µm, and then heated to 80°C and held there for 2 hours. After cooling, fragrance microcapsules were obtained. The mean capsule size was 15.3 µm and the encapsulation efficiency was 99.34%. The capsules were incorporated in fabric conditioner. Cloths washed with this fabric conditioner exhibited enhanced fragrance levels and burst effects compared to cloths washed with neat fragrance, see Figure 1.

### Example III

### Acid catalyst

A reactor was charged with 34g of Alcapsol 144 (Ciba), 18g of Cymel 385 (available from Cytec), and 293g of water. This mixture was stirred until a clear solution with an approximate pH of 6.3 was obtained. Citric acid crystals are added stepwise with dissolving until pH of 5 is reached. This mixture was then stirred for 1 hour at 23°C at which time 210g of the fragrance core consisting of 105g of fragrance accord and 105g of Neobee M-5 oil was added and the mixture high-sheared until a mean droplet size of 8µm was reached. The temperature was raised to 80°C for 2 hours to cure the microcapsules. After cooling a white slurry was obtained. Upon incorporation into fabric conditioner base performance was found to be the same or better than the standard acetic acid catalyzed process, see Figure 2.

### Example IV

### Metal salt catalyst

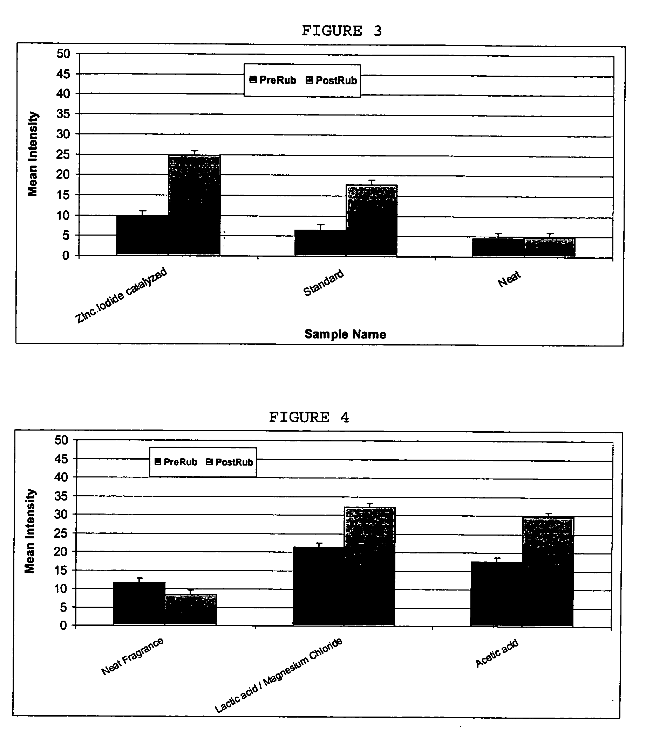

A reactor was charged with 34g of Alcapsol 144 (Ciba), 18g of Cymel 385 (available from Cytec), and 293g of water. This mixture was stirred until a clear solution with an approximate pH of 6.3 was obtained. Acetic acid was added dropwise until pH of 5 was reached. This mixture was then stirred for 1 hour at 23°C at which time 210g of the fragrance core consisting of 105g of fragrance accord and 105g of Neobee M-5 oil was added and the mixture high-sheared until a mean droplet size of 8µm was reached. 2.17g of solid MgCl₂ are added and the dispersion was stirred for 30 minutes to facilitate dissolving of the salt and incorporation into the capsule walls. The temperature was raised to 80 °C for 2 hours to cure the microcapsules. After cooling a white slurry was obtained. Upon incorporation into fabric conditioner base performance was found to be the same or better than the standard acetic acid catalyzed process, see Figure 3.

### Example V

### Acid-metal salt combination

A reactor was charged with 34g of Alcapsol 144 (Ciba), 18g of Cymel 385 (available from Cytec), and 293g of water. This mixture was stirred until a clear solution with an approximate pH of 6.3 was obtained. Citric acid crystals were added stepwise with dissolving until pH of 5 was reached. This mixture was then stirred for 1 hour at 23 °C at which time 210g of the fragrance core consisting of 105g of fragrance accord and 105g of Neobee M-5 oil was added and the mixture high-sheared until a mean droplet size of 8µm was reached. 2.17g of solid MgCl₂ were added and the dispersion was stirred for 30 minutes to facilitate dissolving of the salt and incorporation into the capsule walls. The temperature was raised to 80°C for 2 hours to cure the microcapsules. After cooling a white slurry was obtained. Upon incorporation into fabric conditioner base performance was found to be the same or better than the standard acetic acid catalyzed process, see Figure 4.

### Example VI

### Primary Aminoplast Crosslinking

A reactor was charged with 34g of Alcapsol 144 (Ciba) and 293g of water. A highly reactive crosslinker (Cymel 385) was added in quantities ranging from 10% to 400% the polymer quantity. This mixture was stirred until a clear solution with an approximate pH of 6.3 was obtained. Acetic acid was added until pH 5 was reached. This mixture was then stirred for 1 to 3 hours at 23°C until a Brookfield viscosity of 75 cP is reached. At this point 210g of the fragrance core consisting of 105g of fragrance accord and 105g of Neobee M-5 oil was added and the mixture high-sheared until a mean droplet size of 8µm is reached. The temperature was raised to 80°C for 2 hours to cure the microcapsules. After cooling a white slurry is obtained. Upon incorporation into fabric conditioner base performance was found to be the same or better than the standard acetic acid catalyzed process.

### Example VII

### Primary Aminoplast Crosslinking Blends

A reactor was charged with 34g of Alcapsol 144 (Ciba) and 293g of water. A highly reactive hydrophilic crosslinker (Cymel 385) was added along with a low reactive hydrophobic crosslinker (Cymel 9370). In this case Cymel 385 was needed for wall formation and Cymel 9370 was used for hydrophobicity. The 385:9370 ratio was unlimited so long as there was enough 385 present to cause capsule formation. Typically 385:9370 ratios range from 10:90 to 90:10. This mixture was stirred until a clear solution with an approximate pH of 6.3 was obtained. Acetic acid was added until pH 5 was reached. This mixture was then stirred for 1 hour at 23°C until a Brookfield viscosity of 75 cP was reached. At this point 210g of the fragrance core consisting of 105g of fragrance accord and 105g of Neobee M-5 oil was added and the mixture high-sheared until a mean droplet size of 8µm was reached. The temperature was raised to 80°C for 2 hours to cure the microcapsules. After cooling a white slurry was obtained. Upon incorporation into fabric conditioner base performance was found to be the same or better than the standard acetic acid catalyzed process.

### Example VIII

### Secondary Aminoplast Crosslinking

Standard quantities microcapsule ingredients were combined and made up to the curing stage. Before curing an additional dose of Cymel 385 corresponding to between 50% and 400% times the amount already present in the capsule wall was added, diluted with 2.5 times its weight in water. This second dose of crosslinker was allowed to associate with the capsule wall for one hour at ambient temperature with stirring before being cured as usual. Alternatively the standard capsule was first be cured, followed by the addition of more crosslinker, association time, and a second curing step.

### Example IX

### Secondary Aldehyde Crosslinking

250g of a fragrance microcapsule slurry with amine groups in the capsule walls (polyvinyl amine) was stirred with 1.3 ml of 50% glutaraldehyde solution for 24 hours at room temperature. This results in an amine:crosslinker ratio of 5:1. The resulting slurry was used as is.

## Claims

1. A capsule particle comprising an active material; said active material encapsulated by a polymeric material wherein said polymeric material is selected from the group consisting of an amine-containing polymer, an amine-generating polymer, mixtures thereof and a cross-linker to provide a polymer encapsulated material.

2. The capsule particle of claim 1 wherein the amine-containing polymers comprises primary amine groups; or
comprises secondary amine groups; or
comprises a mixture of primary and secondary amine groups.

3. The capsule particle of claim 1 or claim 2, or the method of claim 25 wherein the polymeric material further comprises polymers with reactive groups selected from the group consisting of COOH, OH, SH and mixtures thereof.

4. The capsule particle of claim 3, or the method of claim 3 wherein the polymers with reactive groups are selected from the group consisting of polyacrylates containing acrylic acid copolymers, polyvinyl alcohol, polysaccharides, cysteine and mixtures thereof.

5. The capsule particle of any preceding claim, or the method of claim 25, or the process of claim 27 wherein the active material is selected from the group consisting of fragrances, flavoring agents, fungicide, brighteners, antistatic agents, wrinkle control agents, fabric softener actives, hard surface cleaning actives, skin conditioning agents, hair conditioning agents, antimicrobial actives, UV protection agents, insect repellants, animal/vermin repellants, flame retardants and mixtures thereof.

6. The capsule particle of any preceding claim, or the process of claim 5 wherein said active material is a fragrance.

7. The capsule particle of any preceding claim wherein said active material further comprises a malodour counteractant composition.

8. The capsule particle of claim 7 wherein said malodour counteractant composition is selected from the group consisting of uncomplexed cyclodextrin, odor blockers, reactive aldehydes, flavanoids, zeolites, activated carbon and mixtures thereof.

9. The capsule particle of any preceding claim, or method of claim 25 or claim 26, or the process of claim 27 wherein the amine-containing polymer is selected from the group consisting of polyvinyl amines, polyvinyl formamides, polyallyl amines, gelatin, zein, albumen, polysaccharides and mixtures thereof.

10. The capsule particle of claims 1 to 8 wherein the polymeric material is of the general formula: 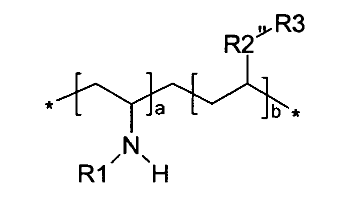 wherein R1 is H, CH₃, (C=O)H, alkylene, alkylene with unsaturated C-C bonds, CH₂-CROH, (C=O)-NH-R, (C=O)-(CH₂)n-OH, (C=O) -R, (CH₂)n-E, -(CH₂-CH(C=O))n-XR, -(CH₂)n-COOH, -(CH₂)n-NH₂, -CH₂)n-(C=O)NH₂, E is an electrophilic group; wherein a and b are integers or averages (real numbers) from about 100-25,000, R is a saturated or unsaturated alkane, dialkylsiloxy, dialkyloxy, aryl, alkylated aryl, and that may further contain a cyano, OH, COOH, NH₂, NHR, sulfonate, sulphate, -NH₂, quaternized amines, thiols, aldehyde, alkoxy, pyrrolidone, pyridine, imidazol, imidazolinium halide, guanidine, phosphate, monosaccharide, oligo or polysaccharide;
R2 can be absent or the functional group selected from the group consisting of -COO-, -(C=O)-, -O-, -S-, -NH-(C=O)-, -NR1-, dialkylsiloxy, dialkyloxy, phenylene, naphthalene, alkyleneoxy; and
R3 can be equal to or selected from the same group as R1.

11. The capsule particle of claims 1 to 8 wherein the polymeric material is of the general formula: 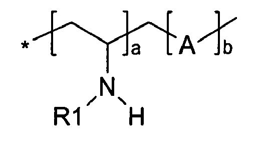 wherein A is an aminal, hydrolyzed or non-hydrolyzed maleic anhydride, vinyl pyrrolidine, vinyl pyridine, vinyl pyridine-N-oxide, methylated vinyl pyridine, vinyl naphthalene, vinyl naphthalene-sulfonate.

12. The capsule particle of claim 11 wherein the aminal is of the general formula: 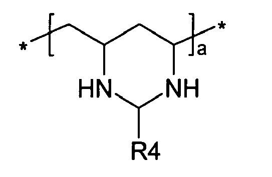 wherein R4 is H, CH₃, (C=O)H, alkylene, alkylene with unsaturated C-C bonds, CH₂-CROH, (C=O)-NH-R, (C=O)-(CH₂)n-OH, (C=O) -R, (CH₂)n-E, - (CH₂-CH(C=O))n-XR, -(CH₂)n-COOH, - (CH₂)n-NH₂, -CH₂)n-(C=O)NH₂ and E is an electrophilic group; wherein a is an integer or average (real number) from about 100-25,000, R is a saturated or unsaturated alkane, dialkylsiloxy, dialkyloxy, aryl, alkylated aryl, and that may further contain a cyano, OH, COOH, NH₂, NHR, sulfonate, sulphate, -NH₂, quaternized amines, thiols, aldehyde, alkoxy, pyrrolidone, pyridine, imidazol, imidazolinium halide, guanidine, phosphate, monosaccharide, oligosaccharide and polysaccharide.

13. The capsule particle of any preceding claim, or method of claim 25 or claim 26 wherein the amine-generating polymer is a polymer comprising vinyl formamides; or the process of claims 27 to 34 wherein the amine-generating polymer is vinyl formamide.

14. The capsule particle of any preceding claim, or the method of claim 25 or claim 26, or the process of claims 27 to 34, wherein the polymer further comprises functional groups selected from the group consisting of imines, amides, enamines, N-nitroso, urea, urethane, ion-paired amine salts, oximes, azo, azoxy, hydrazo groups and mixtures thereof.

15. The capsule particle of any preceding claim, or the method of claim 25 or claim 26, or the process of claims 27 to 34 wherein the crosslinker is selected from the group consisting of aminoplasts, aldehydes, dialdehydes, epoxy, active oxygen, poly-substituted carboxylic acids and derivatives thereof, diketones, halide-substituted, sulfonyl chloride-based organics, inorganic crosslinkers, organic crosslinkers capable of forming azo, azoxy and hydrazo bonds, lactones, lactams, thionyl chloride, phosgene, tannin/tannic acid, polyphenols, free radical crosslinkers and mixtures thereof; or
an aldehyde selected from the group consisting of formaldehyde, acetaldehyde and mixtures thereof; or
glutaraldehyde; or
an active oxygen selected from the group consisting of ozone, OH radicals and mixtures thereof; or
a poly-substituted carboxylic acids and derivatives thereof selected from the group consisting of acid chlorides, anyhydrides, isocyanates and mixtures thereof; or
a calcium ion (Ca²⁺); or
a free radical selected from the group consisting of benzoyl peroxide, sodium persulfate, azoisobutylnitrile (AIBN) and mixtures thereof.

16. The capsule particle of any preceding claim, or the method of claim 25 or claim 26, or the process of claims 27 to 34 wherein the polymer undergoes crosslinking by radiation.

17. The capsule particle of claims 6 to 16 wherein the fragrance materials have greater than about 60 weight percent with ClogP values of equal to or greater than about 3.3.

18. The capsule particle of any preceding claim further comprising a solvent with logP values of greater than about 3.3.

19. The capsule particle of claim 18 wherein the solvent material is selected from the group consisting of triglyceride oil, monoglycerides, diglycerides, mineral oil, silicone oil, diethyl phtalates, polyalpha olefins, isopropyl myristate and mixtures thereof.

20. The capsule particle of any preceding claim wherein the active material is from about 10 to about 50 weight percent of the composition.

21. The capsule particle of any preceding claim which is endcapped with mono-functional aldehydes selected from the group consisting of acetaldehyde, benzaldehyde and mixtures thereof.

22. The capsule particle of any preceding claim, or the method of claim 25 or claim 26 wherein the polymer encapsulated material is further coated with a cationically charged polymer.

23. The capsule particle of any preceding claim which is incorporated into a product selected from the group consisting of a personal care, fabric care and cleaning products.

24. The composition of claim 23 wherein the personal care product is selected from the group consisting of hair shampoos, hair rinses, hair colors and dyes, bar soaps, and body washes.

25. A method of encapsulating an active material comprising:
providing polymeric material wherein said polymeric material is selected from the group consisting of amine-containing polymer, amine generating polymers and mixtures thereof and a primary crosslinker to completion;
encapsulating the active material with the polymeric material to form a polymer encapsulated material;
an optional step of adding a secondary crosslinker to the reacted polymer encapsulated material in an amount sufficient to modify the capsule surface;
an optional step of adding a polymeric material selected from the group consisting of an amine-containing polymer, an amine-generating polymer and mixtures thereof to the encapsulated material to provide a multi-shell morphology around the encapsulated material; and
providing the polymer encapsulated material to a product selected from the group consisting of a personal care, fabric care and cleaning products.

26. The method of claim 25 wherein the active material is a fragrance having greater than about 60 weight percent with clogP values of greater than about 3.3.

27. A process for preparing an encapsulated active material comprising the steps of:
i. reacting a polymer and a primary crosslinker to completion in the presence of a catalyst in an amount sufficient to adjust the pH to a value from about 3 to about 10.
ii. forming a crosslinked network of the polymer and the primary crosslinker;
iii. admixing an active material to the reactant mixture; and
iv. encapsulating the active material with the polymer to form a polymer encapsulated material; and
v. an optional step of adding a secondary crosslinker to the reacted polymer encapsulated material thereby modifying the capsule surface
vi. an optional step of adding a polymer selected from the groups consisting of an amine-containing polymer, an amine-generating polymer and mixtures thereof , to the encapsulated material to provide a multi-shell morphology around the encapsulated material.

28. The process of claim 27 wherein the catalyst is selected from the group consisting of hydrochloric (HCl)acid, sulfuric acid, phosphoric acid, para-toluenesulfonic acid (pTSA), acetic acid, glycolic acid, lactic acid, benzoic acid, citric acid, maleic acid, ammonium chloride, aluminum nitrate, aluminum sulfate, magnesium bromide, magnesium chloride, magnesium nitrate, zinc bromide, zinc iodide, zirconyl nitrate and mixtures thereof.

29. The process of claim 27 or claim 28 wherein the polymer is an acrylamide-acrylic copolymer having a molecular weight in the range of from about 500 to about 5,000,000; and the weight ratio of acrylic acid monomeric units:acrylamide monomeric units is from about 30:1 to about 1:30.

30. The process of claim 29 wherein the weight ratio of primary crosslinker:acrylamide-acrylic acid copolymer is in the range of from about 30:1 to about 1:30; or
from about 14:1 to about 1:14; or
from about 7:1 to about 1:7.

31. The process of claim 29 wherein the mole ratio of acrylic acid monomeric units:acrylamide monomeric units is from about 7:3 to about 3:7.

32. The process of claim 29 wherein the acrylamide-acrylic acid copolymer has a molecular weight in the range of from about 10,000 to about 100,000.

33. The process of claim 27 wherein the polymer is selected from the group consisting of an amine-containing polymer, an amine-generating polymer and mixtures thereof, wherein the polymer has a molecular weight in the range of from about 5,000 to about 1,000,000.

34. The process of claims 27 to 33 wherein the weight ratio of primary crosslinker: amine-containing polymer is in the range of from about 30:1 to about 1:30; or
from about 14:1 to about 1:14; or
from about 7:1 to about 1:7.
